# EUROPEAN PATENT APPLICATION

(11) **EP 2 025 347 A1**
(43) Date of publication of application: **18.02.2009**
(21) Application number: 07743425.6
(22) Date of filing: 09.05.2007
(51) Int. Cl.: A61K 45/00, A61K 31/704, A61K 48/00, A61P 35/00, A61P 43/00

(54) **PROPHYLACTIC AND THERAPEUTIC AGENT FOR CANCER**

(30) Priority: 15.05.2006 JP 2006135886
(71) Applicant: Takeda Pharmaceutical Company Limited, Osaka-shi, Osaka 541-0045 (JP); Sugimoto, Yoshikazu, Kashiwa-shi, Chiba, 2770061 (JP); Katayama, Kazuhiro, Tokyo 1058512 (JP)
(72) Inventor: SUGIMOTO, Yoshikazu, Chiba 2770061 (JP); KATAYAMA, Kazuhiro, Tokyo 1058512 (JP)
(74) Representative: Kalhammer, Georg
(86) International application number: PCT/JP2007/059990
(87) International publication number: WO 2007/132867

(57) **Abstract**

A Ras, Raf, MEK, ERK or RSK inhibitor, namely a P-glycoprotein expression inhibitor or a BCRP expression inhibitor, can be screened by utilizing the MAPK signaling activity as an indicator. It becomes possible to provide an anticancer agent which is reduced in resistance acquisition, and also provide an agent for preventing the resistance against an anticancer agent, which can enhance the therapeutic effect of the anticancer agent against cancer.

## Description

### TECHNICAL FIELD

The present invention relates to a P-glycoprotein or BCRP expression inhibitor and a method of screening the same. The present invention also relates to an anticancer agent which is reduced in acquisition of resistance to anticancer agents. The present invention further relates to an agent for treating cancer which is effective also for cancer which has acquired resistance to anticancer agents.

### BACKGROUND ART

Anticancer drugs of anthracycline type such as doxorubicin, daunorubicin, epirubicin, etc., vinca alkaloids such as vincristine, etc., taxanes such as paclitaxel, docetaxel, etc. have been widely used, as they have extremely potent anti-malignant tumor effects. On the other hand, these anticancer drugs are known to cause adverse effects such as bone marrow suppression, diarrhea, etc. It is also known that anticancer drugs become less effective as a result of acquired resistance of cancers during prolonged administration and some patients might develop resistance to anticancer drugs and hence cannot benefit from chemotherapy.

Mechanism of resistance acquisition to anticancer agents in cancer cells has been studied, and many ABC transporters (membrane proteins having the ATP-binding domain in one molecule and activated by ATP) are known to be involved in the resistance to these anticancer agents.

A P-glycoprotein, which is one of ABC transporters, is known to be involved in anticancer drug resistance (Proc. Natl. Acad. Sci. USA, 84: 3004-3008 (1987)). More specifically, it was found that in cancers expressing a P-glycoprotein, P-glycoprotein excretes anticancer drugs out of the cells to reduce intracellular accumulation of anticancer drugs. A gene encoding this P-glycoprotein is a MDR (multi-drug resistance) 1 gene. It is also known that BCRP (Breast Cancer Resistance Protein) as one of ABC transporters are similarly involved in the anticancer drug resistance (Proc. Natl. Acad. Sci. USA, 95(26), 15665-15670 (1998)).

### DISCLOSURE OF THE INVENTION

However, much less is known about low molecular weight compounds which inhibit the expression of P-glycoprotein in cancer cell lines that have come to acquire anticancer drug resistance as a result of the expression or elevated expression of the ABC transporter, especially P-glycoprotein (Japanese Laid-Open Patent Publication (Tokkai) Nos. 2006-69910 and 2005-247716). It is reported that estrogen downregulates the expression of P-glycoprotein in cultured human breast cancer cell lines (Cancer Res., 65: 596-604, 2005; Proceedings of the 64th Annual Meeting of the Japanese Cancer Association, page 425). Also, much less is known about low molecular weight compounds which inhibit the expression of BCRP in cancer cell lines that have come to acquire anticancer drug resistance as a result of the expression or elevated expression of BCRP (Japanese Laid-Open Patent Publication (Tokkai) No. 2004-123567; Cancer Res. 65: 596-604, 2005).

The present inventors have performed the screening of a variety of materials using cancer cells which express P-glycoprotein among ABC transporters at a high level to find a compound that inhibits the expression ofP-glycoprotein, and has found that MEK and Ras inhibitors markedly inhibit P-glycoprotein. The inventors have further found that ERK and RSK inhibitors also inhibit the expression of P-glycoprotein. The present invention has thus come to be accomplished.

The inventors have further found that the MED inhibitor markedly inhibits the expression of BCRP using cancer cells which express BCRP at a high level.

That is, the present invention provides a P-glycoprotein expression inhibitor comprising a MAPK signaling inhibitor, a method of screening the P-glycoprotein expression inhibitor, an anticancer resistance inhibitor, an agent for treating cancer, and so on, which are described below.

The present invention further provides a BCRP expression inhibitor comprising a MAPK signaling inhibitor, a method of screening the BCRP expression inhibitor, an anticancer resistance inhibitor, an agent for treating cancer, and so on, which are described below.
(1) P-glycoprotein or BCRP expression inhibitor comprising the MAPK signaling inhibitor.
(2) The P-glycoprotein or BCRP expression inhibitor according to (1) above, wherein the MAPK signaling inhibitor is a substance that inhibits the expression of a protein involved in MAPK signaling or a substance that inhibits the activity of a protein involved in MAPK signaling.
(3) The P-glycoprotein or BCRP expression inhibitor according to (1) or (2) above, wherein the MAPK signaling inhibitor is one or at least two selected from a Ras inhibitor, a Raf inhibitor, a MEK inhibitor, an ERK inhibitor and a RSK inhibitor.
(4) The P-glycoprotein or BCRP expression inhibitor according to any one of (1) to (3) above, wherein the MAPK signaling inhibitor is at least one selected from:
   (a) a low molecular weight compound that inhibits the activity of a Ras protein, a Raf protein, a MEK protein, an ERK protein or a RSK protein;
   (b) a low molecular weight compound that inhibits the expression of a Ras protein, a Raf protein, a MEK protein, an ERK protein or a RSK protein;
   (c) siRNA or shRNA for a polynucleotide encoding a Ras protein, a Raf protein, a MEK protein, an ERK protein or a RSK protein; and,
   (d) an antisense polynucleotide comprising the entire or part of a nucleotide sequence complementary or substantially complementary to a nucleotide sequence of a polynucleotide encoding a Ras protein, a Raf protein, a MEK protein, an ERK protein or a RSK protein.
(5) A method of screening a P-glycoprotein or BCRP expression inhibitor using the activity of inhibiting Ras, Raf, MEK, ERK or RSK as an indicator.
(6) An anticancer agent comprising the P-glycoprotein or BCRP expression inhibitor according to (1), which is reduced in resistance acquisition.
(6a) The anticancer agent according to (6) above, comprising the P-glycoprotein expression inhibitor having both an activity of inhibiting the expression of P-glycoprotein and an anticancer activity.
(6b) The anticancer agent according to (6) above, comprising the BCRP expression inhibitor having both an activity of inhibiting the expression of BCRP and an anticancer activity.
(6c) The anticancer agent according to (6) or (6a) above, wherein the P-glycoprotein or BCRP expression inhibitor is the MEK inhibitor.
(7) An agent for preventing the resistance to an anticancer agent comprising the P-glycoprotein or BCRP expression inhibitor according to (1) above.
(8) The agent for preventing the resistance to an anticancer agent according to (7) above, wherein the MAPK signaling inhibitor is a substance that inhibits the expression of a protein involved in MAPK signaling or a substance that inhibits the activity of a protein involved in MAPK signaling.
(9) The agent for preventing the resistance to an anticancer agent according to (7) or (8) above, wherein the MAPK signaling inhibitor is one or at least two selected from a Ras inhibitor, a Raf inhibitor, a MEK inhibitor, an ERK inhibitor and a RSK inhibitor.
(10) An agent for treating cancer comprising a combination of the P-glycoprotein or BCRP expression inhibitor according to (1) above and an anticancer agent.
(11) The agent for treating cancer according to (10) above, wherein the MAPK signaling inhibitor is a substance that inhibits the expression of a protein involved in MAPK signaling or a substance that inhibits the activity of a protein involved in MAPK signaling.
(12) The agent for treating cancer according to (10) or (11)above, wherein the MAPK signaling inhibitor is one or at least two selected from a Ras inhibitor, a Raf inhibitor, a MEK inhibitor, an ERK inhibitor and a RSK inhibitor.
(13) The agent for treating cancer according to any one of (10) to (12) above, wherein the anticancer agent is selected from doxorubicin hydrochloride, daunomycin, epirubicin hydrochloride, an anthracycline, a vinca alkaloid and a taxane.
(14) The method of preventing the expression of P-glycoprotein or BCRP, which comprises administering an effective dose of a MAPK signaling inhibitor.
(15) A method of preventing the expression of P-glycoprotein or BCRP according to (4) above, wherein the MAPK signaling inhibitor is one or at least two selected from a Ras inhibitor, a Raf inhibitor, a MEK inhibitor, an ERK inhibitor and RSK inhibitor.
(16) A method of treating cancer, which comprises administering an effective dose of an anticancer agent comprising the P-glycoprotein or BCRP expression inhibitor according to (1) above.
(17) The method according to (16) above, wherein acquisition of resistance to the anticancer agent is reduced to treat cancer.
(18) The method according to (16) above, wherein excretion of the anticancer agent from a target cancer cell of the anticancer agent is reduced to enhance the therapeutic effect on cancer.

The present invention was presented at the General Meeting of the Japanese Association for Molecular Target Therapy of Cancer (June 15-16, 2006, Tokyo), at the Annual Meeting, Proceedings, of the 65th Japanese Cancer Association (September 28-30, 2006, Yokohama), the Joint Meeting of the 3rd ISC International Conference on Chemotherapeutics and the 11th International Symposium on Cancer Chemotherapy (December 6-8, 2006, Tokyo) and the 127th Annual Meeting of the Pharmaceutical Society of Japan (March 28-30, 2007).

### BRIEF DESCRIPTION OF THE DRAWING

FIG 1 shows inhibition of the expression of endogenous P-glycoprotein by the MAPK signaling or Akt signaling inhibitor.
FIG 2 shows inhibition of the expression of endogenous or exogenous P-glycoprotein by the MEK inhibitor U0126 with passage of time.
FIG. 3 shows inhibition of the expression of endogenous or exogenous P-glycoprotein by the MEK inhibitor PD098059 with passage of time.
FIG 4 shows inhibition of the expression of endogenous or exogenous P-glycoprotein by knockdown of the MEK, ERK, RSK gene with passage of time.
FIG 5 shows inhibition of the expression of endogenous BCRP expression by the MEK inhibitor U0126 with passage of time.
FIG 6 shows increased sensitivity to paclitaxel in cancer cells by the MEK inhibitor.
FIG 7 shows increased uptake of fluorogenic substrate Rhodamine 123 for P-glycoprotein in cancer cells by the MEK inhibitor.

First, the present invention provides the P-glycoprotein or BCRP expression inhibitor comprising a MAPK signaling inhibitor. As used herein, the "MAPK signaling" refers to one of the intracellular signaling pathway which transduces signals from external environment to the interior of cells and is known to be activated by signals such as GPCR (G-protein-coupled receptor), a cell growth factor, a cell differentiation factor, a stress stimulus, etc. The MAPK signaling pathway (MAP kinase cascade) includes cascades through three-tiered kinases of MAPKKK (MAP kinase kinase kinase), MAPKK (MAP kinase kinase) and MAP kinase. In the MAPK signaling pathway, for example, ERK, p38 MAPK, SAPK/JNK, ERK5/BMK, etc. are known as MAPK; MEK, MKK, etc. are known as MAPKK; and Raf, Mos, Tp12, MLK, TAK, DLK, MEKK, ASK, etc are known as MAPKK. In the MAPK signaling pathways, the pathway primarily targeted in the present invention is the pathway where signals are transduced in the order of (1) Raf or Mos (MAPKKK), (2) MEK (MAPKK) and (3) ERK (MAPK).

The "P-glycoprotein" is the protein first identified as an ABC transporter also called MDR1 (Multidrug Resistance 1), which is associated with anticancer drug resistance, and is encoded by MDR1 gene. The sequence of human full-length cDNA of MDR1 gene that is a gene for P-glycoprotein is public known. The MDR1 gene is registered as the accession number M14758 in GenBank and reported in Cell, 47: 381-389 (1986), etc. The gene referred to as human wild-type MDR1 cDNA throughout the specification is the one isolated from human adrenal gland cDNA library (Biochem. Biophys. Res. Commun., 162: 224-231 (1989)).

"BCRP (Breast Cancer Resistance Protein)" is a protein identified as an ABC transporter associated with anticancer drug resistance, which was shown to be expressed in heart tissue among normal tissues and demonstrated to be highly expressed in cancer tissues, especially in breast cancer tissues. BCRP is encoded by a BCRP gene. The sequence of human full-length cDNA of BCRP gene which is a gene for BCRP is public known. The BCRP gene is registered as accession number AB056867 in GenBank and reported in Doyle, L.A., Yang, W., Abruzzo, L. V, Krogmann, T., Gao, Y, Rishi, A.K. and Ross, D. D., A multidrug resistance transporter from human MCF-7 breast cancer cells, Proc. Natl. Acad. Sci. U.S.A., 95(26), 15665-15670 (1998), etc.

"Resistance to anticancer agents/anticancer drug resistance" refer to a state where a reduction or loss in the pharmacological effects occurs in the treatment or preventive treatment of cancer, as a result of the repeated administration of anticancer drugs or as a result of the congenitally upregulated expression of P-glycoprotein or BCRP.

"Acquisition of resistance to anticancer agents/anticancer drug resistance" refers to a state where the sensitivity to anticancer drugs or their analogues is diminished, as a result of the repeated administration of anticancer drugs, or a state where the sensitivity to anticancer drugs or their analogues is reduced, as a result of the congenitally upregulated expression or acquired overexpression of P-glycoprotein or BCRP for some reasons.

The present inventors have found that the expression of P-glycoprotein or BCRP can be suppressed by MAPK signaling inhibitors (especially, inhibitors that inhibit the signal transduction of cascades involving Raf, Mos, MEK and ERK).

The MAPK signaling inhibitor refers to a substance that inhibits the signal transduction as a result of inhibition of expression, inhibition of activation, destabilization, etc. of *in vivo* molecules including Ras, MOS, TpI2, Raf, MEK, ERK, RSK, MKK, p38 MARK, SAPK, JNK, MLK, MEKK, MLK, ASK, etc., which are involved in MAPK signaling. The inhibition of signals includes both complete interruption and weakening of signals. As such substances, not only low molecular weight or high molecular weight compounds but also siRNA, shRNA, an antibody, an antisense, a peptide, a protein, an enzyme, etc. can be used.

Examples of the MAPK signaling inhibitor which can be used include a substance that inhibits the expression of a protein involved in MAPK signaling, a substance that inhibits the activity of a protein involved in MAPK signaling (including translation products of genes involved in MAPK signaling), etc. As used herein, the term "inhibit the expression of a protein" means inhibition of the production of said protein by inhibiting any one of a series of events up to the production of the protein from a gene encoding the protein (including, e.g., transcription (production of mRNA), translation (production of the protein)), including the inhibition of expression of the gene for the protein.

The term "inhibit the activity of a protein" means inhibition of the physiological functions possessed by said protein (for example, the subsequent protein phosphorylation in the MAPK signaling pathway) and further includes inhibition of the event to transform said protein to its active form (e.g., phosphorylation) to suppress the physiological functions of said protein by destabilization (or inhibition of stabilization), etc.

The protein involved in MAPK signaling (for example, Ras protein, Raf protein, MEK protein, ERK protein, RSK protein, etc.) is sometimes referred to as the protein used in the present invention. The gene involved in MAPK signaling (including a gene encoding the protein involved in MAPK signaling) is sometimes referred to as the gene used in the present invention.

The MAPK signaling inhibitor used in the present invention includes, for example, (a) a low molecular weight compound that inhibits the activity of the protein used in the present invention, (b) a low molecular weight compound that inhibits the expression of a protein used in the present invention, (c) a siRNA or shRNA to a polynucleotide encoding the protein used in the present invention, (d) an antisense polynucleotide comprising an entire or part of a nucleotide sequence complementary or substantially complementary to the nucleotide sequence of a polynucleotide encoding the protein used in the present invention, (e) an antibody against the protein used in the present invention, (f) a ribozyme to a polynucleotide encoding the protein used in the present invention, (g) a variant of the protein used in the present invention acting dominant-negatively against the protein used in the present invention or a polynucleotide encoding the same, (h) an aptamer to the protein used in the present invention, etc.

The substance that inhibits the expression of the protein used in the present invention is not particularly limited so far as the substance inhibits the expression of the protein used in the present invention and includes, for example, (i) a substance that inhibits the transcription from a gene (DNA) encoding the protein used in the present invention into an mRNA encoding the protein used in the present invention, (ii) a substance that inhibits the translation from an mRNA encoding the protein used in the present invention into the protein used in the present invention, etc. (i) The substance that inhibits the transcription from a gene (DNA) encoding the protein used in the present invention into an mRNA encoding the protein used in the present invention is not particularly limited so far as the substance inhibits the transcription from a gene (DNA) encoding the protein used in the present invention into an mRNA encoding the protein used in the present invention and includes, for example, a substance that binds to a factor associated with the transcription from a gene (DNA) encoding the protein used in the present invention into an mRNA to inhibit the transcription from a gene (DNA) encoding the protein used in the present invention into an mRNA, etc. (ii) The substance that inhibits the translation from an mRNA encoding the protein used in the present invention to the protein used in the present invention is not particularly limited so far as the substance inhibits the translation from an mRNA encoding the protein used in the present invention into the protein used in the present invention and includes, for example, a substance that binds to a factor associated with the translation from an mRNA encoding the protein used in the present invention to the protein used in the present invention to inhibit the translation from an mRNA encoding the protein used in the present invention into the protein used in the present invention, etc. Specific examples of the substance that inhibits the expression of the protein used in the present invention include (b) a low molecular weight compound that inhibits the expression of the protein used in the present invention, (c) a siRNA or shRNA to a polynucleotide encoding the protein used in the present invention, (d) an antisense polynucleotide comprising an entire or part of a nucleotide sequence complementary or substantially complementary to the nucleotide sequence of a polynucleotide encoding an protein used in the present invention, (f) a ribozyme to a polynucleotide encoding the protein used in the present invention, etc.

The substance that inhibits the activity of the protein used in the present invention is not particularly limited so far as the substance inhibits the activity of the protein used in the present invention and includes, for example, (i) a substance that inhibits the activation of a protein involved in MAPK signaling, (ii) a substance wherein a protein involved in MAPK signaling inhibits the activation of a downstream factor (e.g., a protein) of the protein. As used herein, the term "inhibit the activation of a protein involved in MAPK signaling" means to include direct inhibition of the event to transform a protein involved in MAPK signaling into its active form (e.g., phosphorylation), but is not limited thereto. The inhibition of the activation of a protein involved in MAPK signaling further includes, for example, to inhibit the activation of a protein involved in MAPK signaling by inhibiting the expression or activation of a upstream factor (e.g., a protein) of the MAPK signaling system or destabilizing the same (or inhibiting its stabilization).

More specifically, the MAPK signaling inhibitor which can be used includes, for example, a Ras inhibitor, a Raf inhibitor, a MEK inhibitor, an ERK inhibitor, a RSK inhibitor, etc. These inhibitors can be used solely or in combination of two or more, depending upon necessity.

Examples of the Ras inhibitor are R115777 (Zarnesta), BMS-214662, SCH66336, L-778, 123, etc., in addition to the drugs described in EXAMPLES (FTI-277). R115777, BMS-214662 and SCH66336 are farnesyltransferase inhibitors, and L-778 and 123 are geranylgeranyltransferase inhibitors. These inhibitors inhibit the activation of Ras since a low molecular weight G protein of the Ras superfamily inhibits modifications (farnesylation and geranylgenranylation) necessary to exhibit the function through binding to membrane structures of cells. FTI-277 is a farnesyltransferase inhibitor (generic name: N-[4-[2-(R)-amino-3-mercaptopropyl]amino-2-phenyl-benzoyl]methionine) and available from EMD Bioscience, Inc. (Cancer Res., 59: 4919-4926 (1999)).

The Raf inhibitor includes, for example, Bay43-9006 (selective inhibitor for B-Raf and C-Raf; also called sorafenib), etc. Preferred examples of the Raf inhibitor are A-Raf, B-Raf and C-Raf inhibitors; among others, B-Raf inhibitor is particularly preferred.

The MEK inhibitor includes PD184161 (selective inhibitor for MEK1 and MEK2), etc., in addition to the drug (U0126, PD098059) described in EXAMPLES. U0126 is a selective inhibitor for MEK1/2 (generic name: 1,4-diamino-2,3-dicyano-1,4-bis[2-aminophenylthio]butadiene). U0126 which is an organic compound (1,4-diamino-2,3-dicyano-1,4-bis[2-aminophenylthio]butadiene) produced by chemical synthesis inhibits the MAPKK (MEK) activity to suppress the activation of ERK 1/ERK 2. This compound inhibits both phosphorylation of MEK 1/2 by Raf and phosphorylation of ERK 1/2 by MEK 1/2 when compared to PD098059, so that the compound can effect the inhibition more efficiently (J. Biol. Chem., 273: 18623-18632 (1998)). U0126 is available from Cell Signaling Technology, Inc. PD098059 is a selective inhibitor for MEK1 (generic name: 2'-amino-3'-methoxyflavone) and available from Promega Corp. PD098059 is a potent and selective inhibitor for MAPK/ERK kinase I (MAP kinase kinase I or MEK 1) having cell permeability, and blocks the activation of MEK1 to inhibit the next phosphorylation/activation of MAPK kinase (Exp. Cell Res., 253, 255-270(1999)).

The MEK inhibitor includes drugs described in PCT International Publication Pamphlet WO 9837881, PCT International Publication Pamphlet WO 99901426, Japanese Laid-Open Patent Publication (Tokkai) No. 2001-55376, PCT International Publication Pamphlet WO 200041505, PCT International Publication Pamphlet WO 200041994, PCT International Publication Pamphlet WO 200042002, PCT International Publication Pamphlet WO 200042003, PCT International Publication Pamphlet WO 200042022, PCT International Publication Pamphlet WO 200042029, PCT International Publication Pamphlet WO 200056706, PCT International Publication Pamphlet WO 200068199, PCT International Publication Pamphlet WO 200068200, PCT International Publication Pamphlet WO 200068201, PCT International Publication Pamphlet WO 200168619, PCT International Publication Pamphlet WO 200236570, etc. Among others, MEK1 and MEK2 inhibitors are preferred.

The ERK inhibitor includes, for example the inhibitor described in Japanese Laid-Open Patent Publication (Tokkai) No. 2005-330265, RK inhibitor (Merck Calbiochem), 5-Iodotubercidin (Merck Calbiochem), etc. The ERK inhibitor (Merck Calbiochem) is an ERK2-specific inhibitor (KD = -5 mM) (generic name: 3-(2-aminoethyl)-5-[(4-ethoxyphenyl)methylene]-2, 4-thiazolidinedione). 5-Iodotubercidin (Merck Calbiochem) is an ERK2-competitive inhibitor (Ki = 530 nM) (generic name:
4-amino-5-iodo-7-(β-D-ribofuranosyl)pyrrolo[2,3-d]-pyrimidine)and is known to also inhibit adenosine kinase (Ki = 30 nM). Among others, ERK1 and ERK2 inhibitors are preferred.

The RSK inhibitor includes, for example, Kaempherol-3-O-(4'-O-acetyl-a-L-rhamnopyranoside), etc. RSK1, RSK2 and RSK3 inhibitors are preferred as the RSK inhibitor.

Examples of the MAPK signaling inhibitor, which can be preferably employed, are (a) a low molecular weight substance that inhibits the activity of Ras protein, Raf protein, MEK protein, ERK protein or RSK protein, (b) a low molecular weight substance that inhibits the expression of Ras protein, Raf protein, MEK protein, ERK protein or RSK protein, (c) a siRNA or shRNA to a polynucleotide encoding the Ras protein, Raf protein, MEK protein, ERK protein or RSK protein, (d) an antisense polynucleotide comprising an entire or part of a nucleotide sequence complementary or substantially complementary to the nucleotide sequence of a polynucleotide encoding Ras protein, Raf protein, MEK protein, ERK protein or RSK protein, (e) an antibody against Ras protein, Raf protein, MEK protein, ERK protein or RSK protein, (f) a ribozyme to Ras protein, Raf protein, MEK protein, ERK protein or RSK protein; etc. These substances can be employed alone or, if necessary, in combination of two or more.

The term "low molecular weight substance" is used to mean an organic or inorganic substance having a molecular weight of 10, 000 or less (preferably, a molecular weight of 5, 000 or less, more preferably, a molecular weight of 2, 000 or less, most preferably, a molecular weight of 700 or less).

The Ras protein is a GTP-binding protein having the function to transduce signals to MAPKKK as a Raf protein in the MAPK signaling pathway; K-Ras, N-Ras, H-Ras, etc. are known as the Ras protein and described in, e.g., Leukemia, 17: 1263-1293 (2003). The activity of Ras protein can be determined by a method described in, e.g., J. Biol. Chem., 277: 7865-7874 (2002) or its modifications.

The Raf protein as MAPKKK is a kinase having the function to transduce signals to the MEK protein, which is MAPKK, and includes A-Raf, B-Raf, C-Raf, etc., which is described in, e.g., Leukemia 17: 1263-1293 (2003). The activity of Raf protein can be determined by the method described in, e.g., Methods Enzymol., 255: 279-290 (1995), or its modifications.

The MEK protein as MAPKK is a kinase having the function to transduce signals to the ERK protein, which is MAPK, and includes MEK1, MEK2, etc., which is described in, e.g., Leukemia, 17: 1263-1293 (2003). The activity of MEK protein can be determined by the method described in, e.g., Science, 258: 478-480 (1992), or its modifications.

The ERK protein is a MAP kinase having the function to upregulate gene expression through phosphorylation of serine-threonine residues in various transcription factors and includes ERK1, ERK2, etc., which is described in, e.g., World J. Gastroenterol., 2006 Apr 21; 12(15): 2445-2449: Related Articles, Links, etc. The activity of ERK protein can be determined by the methods described in, e.g., World J. Gastroenterol., 2006 Apr 21; 12(15), etc., or their modifications.

The RSK protein is a MAP kinase activated protein kinase (MAPKAP kinase) and described in, e.g., Leukemia, 17: 1263-1293 (2003). The activity of RSK protein can be determined by the method described in, e.g., EMBO J., 14: 674-684 (1995), or its modifications.

The activity of the protein used in the present invention as used herein is sometimes referred to as the activity to transduce signals to a protein at a subsequent step in the MAPK signaling pathway (e.g., phosphorylation activity; hereinafter also referred to as "signaling activity in the MAPK signaling pathway").

The term "Ras protein, Raf protein, MEK protein, ERK protein or RSK protein" as used herein further includes their variants as long as they have substantially the same activities as those of the proteins. The variants of the proteins described above include, for example, proteins having amino acid sequences, wherein 1 or at least 2 (e.g., approximately 1 to 30, preferably approximately 1 to 10, more preferably several (1 to 5)) amino acids are deleted, substituted, added and/or inserted in the amino acid sequences described in the literatures *supra*. Where insertion, deletion or substitution occurs in an amino acid sequence, positions for the insertion, deletion or substitution are not particularly limited.

As the activity of substantially the same nature, there are, for example, a MAPK signaling activity, and the like. The term "substantially the same nature" is used to mean that the nature of these activities is equivalent in terms of quality (e.g., physiologically or pharmacologically). It is thus preferred that fatty acid synthase activities, etc. are equivalent (e.g., approximately 0.01 to 100 times, preferably approximately 0.5 to 10 times, more preferably 0.5 to 2 times), but differences in quantitative factors such as a level of these activities, or such as a molecular weight of the protein may be present and allowable.

The partial peptide of the protein used in the present invention may be any peptide as long as it is a partial peptide of the protein used in the present invention described above and preferably has the property equivalent to that of the protein used in the present invention described above.

For example, in the constituent amino acid sequence of protein used in the present invention, peptides containing, e.g., at least 20, preferably at least 50, more preferably at least 70, much more preferably at least 100 and most preferably at least 200 amino acids, can be used.

The partial peptide used in the present invention may be peptides containing the amino acid sequence, of which at least 1 or 2 (preferably about 1 to about 20, more preferably about 1 to about 10 and most preferably several (1 to 5)) amino acids may be deleted; peptides containing the amino acid sequence, to which at least 1 or 2 (preferably about 1 to about 20, more preferably about 1 to about 10 and most preferably several (1 to 5)) amino acids may be added; peptides containing the amino acid sequence, in which at least 1 or 2 (preferably about 1 to about 20, more preferably about 1 to about 10 and most preferably several (1 to 5)) amino acids may be inserted; or peptides containing the amino acid sequence, in which at least 1 or 2 (preferably about 1 to about 20, more preferably several and most preferably about 1 to about 5) amino acids may be substituted by other amino acids.

The partial peptide of the present invention or its salts can be prepared by publicly known methods for peptide synthesis, or the partial peptide can be prepared by cleaving the protein with an appropriate peptidase. For the methods for peptide synthesis, for example, either solid phase synthesis or liquid phase synthesis may be used. That is, the partial peptide or amino acids that can constitute partial peptide used in the present invention are condensed with the remaining part. Where the product contains protecting groups, these protecting groups are removed to give the desired peptide. Publicly known methods for condensation and elimination of the protecting groups are described in (i) - (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the peptide of the present invention. When the peptide obtained by the above methods is in a free form, the peptide can be converted into an appropriate salt by a publicly known method; conversely when the peptide is obtained in a salt form, it can be converted into its free form by publicly known methods.

The inhibitory activity against the expression ofP-glycoprotein can be determined by measuring the expression level ofP-glycoprotein by the western blotting assay in EXAMPLES later described. Also, the inhibitory activity against the expression of BCRP can be determined by measuring the expression level of BCRP by the western blotting assay in EXAMPLES later described.

In addition, the inhibitory activity can also be determined by FACS. According to FACS (fluorescence activated cell sorting), P-glycoprotein that would be expressed on the cell surface is stained using a labeled antibody, and the stained cells are guided in a liquid flow to measure the amount of the label (level of P-glycoprotein). Specifically, for example, biotinylated P- glycoprotein antibody (MRK16) is reacted with the P-glycoprotein expressed on the cell surface and then reacted with PE-labeled streptoavidin; and the level ofP-glycoprotein expressed on the cell surface can be determined by measuring the brightness ofPE.

The expression level of BCRP can be determined by FACS (fluorescence activated cell sorting). BCRP that would be expressed on the cell surface is stained using a labeled antibody, and the stained cells are guided in a liquid flow to measure the amount of the label (level of BCRP). Specifically, for example, biotinylated BCRP antibody is reacted with the BCRP expressed on the cell surface and then reacted with PE-labeled streptoavidin; and the level of BCRP expressed on the cell surface can be determined by measuring the brightness of PE.

### (a) Low molecular weight compound that inhibits the activity of Ras protein, Raf protein, MEK protein, ERK protein or RSK protein

The compound or its salts can inhibit the activity of the protein used in the present invention, and is thus preferably used as a substance that inhibits the activity of the protein used in the present invention. The compound or its salts that inhibit the activity of the protein used in the present invention are not particularly limited so far as the compound or its salts can inhibit the activity possessed by the protein used in the present invention (e.g., MAPK signaling activity), and includes, for example, a compound or its salts and the like that bind to the protein used in the present invention to inhibit the activity. Examples of these compound or its salts may be those selected from peptides, proteins, non-peptide compounds, synthetic compounds, fermentation products, cell extracts, plant extracts, animal tissue extracts, plasma, etc. These compounds may be novel or publicly known compounds. As salts of the compound, there are, for example, physiologically acceptable metal salts, ammonium salts, salts with organic bases, salts with inorganic acids, salts with organic acids, salts with basic or acidic amino acids, etc. Preferred examples of the metal salts include alkali metal salts such as sodium salts, potassium salts, etc.; alkaline earth meal salts such as calcium salts, magnesium salts, barium salts, etc.; aluminum salts, etc. Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Preferred examples of the salts with inorganic acids include salts with hydrochloric acid, hydrobromic acid, nitric acid, sulfuric acid, phosphoric acid, etc. Preferred examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of the salts with basic amino acids include salts with arginine, lysine, ornithine, etc., and preferred examples of the salts with acidic amino acids include salts with aspartic acid, glutamic acid, etc.

Among others, physiologically acceptable salts are preferred. For example, where the compounds contain acidic functional groups therein, examples include inorganic salts such as alkali metal salts (e.g:, sodium salts, potassium salts, etc.), alkaline earth metal salts (e.g., calcium salts, magnesium salts, barium salts, etc.), ammonium salts, etc., and when the compounds contain basic functional groups therein, examples include salts with inorganic acids such as hydrobromic acid, nitric acid, sulfuric acid, phosphoric acid, etc., salts with organic acids such as acetic acid, phthalic acid, fumaric acid, oxalic acid, tartaric acid, maleic acid, citric acid, succinic acid, methanesulfonic acid, p-toluenesulfonic acid, etc.

Examples of these compounds are Ras inhibitor, Raf inhibitor, MEK inhibitor, ERK inhibitor, RSK inhibitor, etc. Also, these compounds can be obtained by the screening methods described later.

### (b) Low molecular weight compound that inhibits the expression of Ras protein, Raf protein, MEK protein, ERK protein or RSK protein

The compound or its salts can inhibit the expression of the protein used in the present invention, and is thus preferably used as substances that inhibit the expression of the protein used in the present invention. The compound or its salts that inhibit the expression of the protein used in the present invention are not particularly limited so far as the compound or its salts that inhibit the expression of the protein used in the present invention, and include, for example, (i) a compound that inhibits the transcription from a gene (DNA) encoding the protein used in the present invention into an mRNA encoding the protein used in the present invention, (ii) a substance that inhibits the translation from an mRNA encoding the protein used in the present invention into the protein used in the present invention, etc. (i) The compound that inhibits the transcription from a gene (DNA) encoding the protein used in the present invention into an mRNA encoding the protein used in the present invention is not particularly limited so far as the compound inhibits the transcription from a gene (DNA) encoding the protein used in the present invention into an mRNA encoding the protein used in the present invention, and includes, for example, a compound that binds to a factor associated with the transcription from a gene (DNA) encoding the protein used in the present invention into an mRNA and inhibits the transcription from a gene (DNA) encoding the protein used in the present invention into an mRNA, etc. (ii) The compound that inhibits the translation from an mRNA encoding the protein A used in the present invention to the protein used in the present invention is not particularly limited so far as the compound inhibits the translation from an mRNA encoding the protein A used in the present invention into the protein used in the present invention, and includes, for example, a compound that binds to a factor associated with the translation from an mRNA encoding the protein A used in the present invention into the protein used in the present invention and inhibits the translation from an mRNA encoding the protein used in the present invention into the protein used in the present invention, etc.

The Ras inhibitor, Raf inhibitor, MEK inhibitor, ERK inhibitor, RSK inhibitor, etc. are included as these compounds. Also, these compounds can be obtained by the screening methods described later.

### (c) siRNA or shRNA to polynucleotide encoding the Ras protein, Raf protein, MEK protein, ERK protein or RSK protein

The double-stranded RNA having RNAi action on the polynucleotide encoding the protein used in the present invention (e.g., siRNA or shRNA, etc. to the polynucleotide encoding the protein used in the present invention) is low toxic and can suppress the translation of a gene encoding the protein used in the present invention to downregulate the expression of the protein used in the present invention, and thus can be used advantageously as the substance that inhibit the expression of the protein used in the present invention. The double-stranded RNA having RNAi action on the polynucleotide encoding the protein used in the present invention as described above includes a double-stranded RNA containing a part of RNA encoding the protein used in the present invention (e.g., a siRNA (small (short) interfering RNA), a shRNA (small (short) hairpin RNA), etc.

These double stranded RNAs can be manufactured by designing the same based on the sequence of the polynucleotide of the present invention, by a modification of publicly known methods (e.g., Nature, 411, 494, 2001; Japanese National Publication (Tokuhyo) No. 2002-516062; U. S. Patent Application No. 2002/086356; Nature Genetics, 24, 180-183, 2000; Genesis, 26, 240-244, 2000; Nature, 407, 319-320, 2002; Genes & Dev., 16, 948-958, 2002; Proc. Natl. Acad. Sci. USA., 99, 5515-5520, 2002; Science, 296, 550-553, 2002; Proc. Natl. Acad. Sci. USA, 99, 6047-6052, 2002; Nature Biotechnology; 20, 497-500, 2002; Nature Biotechnology, 20, 500-505, 2002; and Nucleic Acids Res., 30, e46, 2002).

The length of double stranded RNA having RNAi action used in the present invention is usually 17 to 30 nucleotides, preferably 19 to 27 nucleotides, and more preferably 20 to 22 nucleotides.

### (d) Antisense polynucleotide having a complementary or substantially complementary nucleotide sequence to the nucleotide sequence of the polynucleotide encoding the Ras protein, Raf protein, MEK protein, ERK protein or RSK protein

The antisense polynucleotide having a complementary or substantially complementary nucleotide sequence to the nucleotide sequence of the polynucleotide encoding the protein or partial peptide used in the present invention (preferably, DNA) (hereinafter these DNAs are sometimes collectively referred to as the DNA of the present invention in the description of antisense polynucleotide) can be any antisense polynucleotide, so long as it possesses the entire or a part of the nucleotide sequence complementary or substantially complementary to the nucleotide sequence of the DNA used in the present invention and is capable of suppressing the expression of said DNA, but preferred is antisense DNA.

The nucleotide sequence substantially complementary to the DNA as used in the present invention includes, for example, a nucleotide sequence having at least about 70% homology, preferably at least about 80% homology, more preferably at least about 90% homology and most preferably at least about 95% homology, to the entire nucleotide sequence or to its partial nucleotide sequence of the DNA used in the present invention (i.e., complementary strand to the DNA used in the present invention), and the like. Especially in the entire nucleotide sequence of the complementary strand to the DNA used in the present invention, preferred are (i) an antisense polynucleotide having at least about 70% homology, preferably at least about 80% homology, more preferably at least about 90% homology and most preferably at least about 95% homology, to the complementary strand of the nucleotide sequence which encodes the N-terminal region of the protein used in the present invention (e.g., the nucleotide sequence around the initiation codon) in the case of antisense polynucleotide directed to translation inhibition and (ii) an antisense polynucleotide having at least about 70% homology, preferably at least about 80% homology, more preferably at least about 90% homology and most preferably at least about 95% homology, to the complementary strand of the entire nucleotide sequence of the DNA used in the present invention including intron, in the case of antisense polynucleotide directed to RNA degradation by RNaseH, respectively.

The antisense polynucleotide is composed of nucleotides of generally about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The nucleotide part (pyrimidine, purine) may also be chemically modified and may be any one which hybridizes to a DNA containing the nucleotide sequence represented by SEQ ID NO: 2, etc. These antisense polynucleotides may be synthesized using a publicly known DNA synthesizer, etc.

The antisense polynucleotide of the present invention may contain altered or modified sugars, bases or linkages, may be provided in a specialized form such as liposomes, microspheres, or may be applied to gene therapy, or may be provided in combination with attached moieties. Such attached moieties used include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol, and the like.

The inhibitory activity of the antisense polynucleotide can be investigated using the transformant of the present invention, the *in vivo* or *in vitro* gene expression system of the present invention, or the *in vivo* or *in vitro* translation system of the protein used in the present invention.

### (e) Antibody against the Ras protein, Raf protein, MEK protein, ERK protein or RSK protein (hereinafter, also referred to as "the protein used in the present invention")

The antibodies against the protein used in the present invention, its partial peptide or its salts may be either polyclonal antibodies or monoclonal antibodies, as long as they are antibodies capable of recognizing antibodies against the protein used in the present invention, its partial peptide or its salts.

The antibodies against the protein used in the present invention, its partial peptide or its salts (hereinafter, sometimes merely referred to as the protein used in the present invention in the description of the antibodies) can be produced by a publicly known method of producing an antibody or antiserum, using as an antigen the protein used in the present invention.

### [Preparation of monoclonal antibody]

### (i) Preparation of monoclonal antibody-producing cells

The protein used in the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mouse, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (ii) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody].

### [Preparation of polyclonal antibody]

The polyclonal antibody of the present invention can be manufactured by publicly known methods or modifications thereof For example, a warm-blooded animal is immunized with an immunogen (protein antigen) per se, or a complex of immunogen and a carrier protein is formed and the animal is immunized with the complex in a manner similar to the method described above for the manufacture of monoclonal antibodies. The product containing the antibody against the protein used in the present invention is collected from the immunized animal followed by separation and purification of the antibody.

In the conjugate of immunogen and a carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out according to the method for the separation and purification of immunoglobulins performed as in the separation and purification of monoclonal antibodies described above.

### (f) Ribozyme to the polynucleotide encoding the Ras protein, Raf protein, MEK protein, ERK protein or RSK protein

The polynucleotide having a ribozyme activity against the polynucleotide encoding the protein used in the present invention can downregulate the expression of the protein used in the present invention, and is thus preferably used as the substance that inhibits the expression of the protein used in the present invention. These ribozyme can be manufactured by designing the same based on the sequence of the polynucleotide of the present invention, by a modification of publicly known methods (e.g., TRENDS in Molecular Medicine, 7, 221, 200; FEBS Lett., 228, 228, 1988; FEBS Lett., 239, 285, 1988; Nucl. Acids. Res., 17, 7059, 1989; Nature, 323, 349, 1986; Nucl. Acids. Res., 19, 6751, 1991; Protein Eng., 3, 733, 1990; Nucl. Acids Res., 19, 3875, 1991; Nucl. Acids Res., 19, 5125, 1991; Biochem. Biophys. Res. Commun., 186, 1271, 1992, etc.). For example, the ribozyme can be manufactured by ligating a publicly known ribozyme to a part of the RNA encoding the protein used in the present invention. The part of the RNA encoding the protein used in the present invention includes a contiguous part (RNA fragment) to the cleavage site on the RNA of the present invention, which can be cleaved by a publicly known ribozyme. The ribozymes described above include large ribozymes such as the group I intron-type or the M1 RNA contained in RNaseP, small ribozymes such as the hammerhead-type or the hairpin-type, etc. (Protein, Nucleic acid, and Enzyme, 35, 2191, 1990). For the hammerhead-type ribozymes, reference can be made on, e.g., FEBS Lett., 228, 228, 1988; FEBS Lett., 239, 285, 1988; Protein, Nucleic Acid, Enzyme, 35, 2191, 1990; Nucl. Acids Res., 17, 7059, 1989, etc. For the hairpin-type ribozymes, reference can be made on, e.g., Nature, 323, 349, 1986; Nucl. Acids Res., 19, 6751, 1991; KAGAKU-TO-SEIBUTSU, 30, 112, 1992; etc.

### (g) Variant of the protein used in the present invention acting dominant negatively against the protein used in the present invention or the polynucleotide encoding the same

The variant of the protein used in the present invention acting dominant negatively against the protein used in the present invention or the polynucleotide encoding the same can inhibits the activity of the protein used in the present invention, and hence can be preferably used as the substance that inhibits the activity of the protein used in the present invention. As used herein, the term "variant of the protein acting dominant negatively against the protein used in the present invention" means a protein having an action to inhibit (eliminate or diminish) the activity of the protein used in the present invention through expression of the variant (cf., IDENSHI-NO-KINO SOGAI JIKKEN-HO (Experimental Technique for Inhibiting Gene Function) edited by Kazunari Taira, YODOSHA Publishing Co., 26-32, 2001., etc.).

### (h) Aptamer to the protein used in the present invention

The aptamer against the protein used in the present invention can inhibits the activity or function of the protein used in the present invention, and is thus preferably used as the substance that inhibits the activity of the protein used in the present invention. The aptamer is obtained by publicly known methods, for example, SELEX (systematic evolution of ligands by exponential enrichment) (Annual Review of Medicine, 56, 555-583, 2005). Structure of the aptamer can be determined using publicly known methods, and the aptamer is produced in accordance with methods publicly known, based on the structure determined.

### (P-glycoprotein expression inhibitor and the agent for preventing the resistance to an anticancer agent)

### (BCRP Expression inhibitor and the agent for preventing the resistance to an anticancer agent)

In the present invention, the active ingredients of (a) the antibody against the protein used in the present invention, (b) the antisense polynucleotide comprising an entire or part of a nucleotide sequence complementary or substantially complementary to the nucleotide sequence of a polynucleotide encoding an protein used in the present invention, (c) the siRNA or shRNA to a polynucleotide encoding the protein used in the present invention, (d) the ribozyme to the polynucleotide encoding the protein used in the present invention, (e) the low molecular weight compound that inhibits the activity of the protein used in the present invention, (f) the low molecular weight compound that inhibits the expression of the protein used in the present invention, etc. can be subjected to pharmaceutical manufacturing processes in a conventional manner, and can be administered as the P-glycoprotein expression inhibitor or the BCRP expression inhibitor. The P-glycoprotein expression inhibitor can suppress the expression of P-glycoprotein thereby to suppress the excretion of anticancer drugs from the target cancer. Accordingly, the P-glycoprotein expression inhibitor can be used as the agent for preventing the resistance to anticancer agents. The BCRP expression inhibitor can suppress the expression of P-glycoprotein thereby to suppress the excretion of anticancer drugs from the target cancer. Thus, the BCRP expression inhibitor can be used as the agent for preventing the resistance to anticancer agents.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injection, etc. These injectable preparations may be prepared by methods publicly known, e.g., by dissolving, suspending or emulsifying the active ingredients described above in a sterile aqueous medium or oily medium conventionally used for injections. As the aqueous medium for injections, there are, for example, physiological saline, an isotonic solution containing glucose and other auxiliary agents, etc., which may be used in combination with an appropriate solubilizing aid such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing aid such as benzyl benzoate, benzyl alcohol, etc. The injection thus prepared is preferably filled in an appropriate ampoule. The suppository used for rectal administration may be prepared by blending the aforesaid substance with conventional bases for suppositories.

Each composition described above may further contain other active ingredients unless formulation causes any adverse interaction with the active ingredients described above.

A dose of the active ingredient may vary depending upon its effects, target disease, subject to be administered, conditions, route of administration, etc.: in oral administration, the active ingredient is generally administered to an adult (as 60 kg body weight) for the purpose of preventing the resistance against an anti-lung cancer agent in a daily dose of about 0.01 to about 100 mg, preferably about 0.1 to about 50 mg and more preferably about 0.1 to about 20 mg. In parenteral administration, a single dose of the active ingredient may vary depending upon the target disease, subject to be administered, conditions, route of administration, etc.; in the form of an injectable dosage form, it is advantageous to administer the active ingredient to an adult (as 60 kg body weight) for the purpose of preventing the resistance against an anti-lung cancer agent generally in a daily dose of about 0.01 to about 30 mg, preferably about 0.1 to about 20 mg, and more preferably about 0.1 to about 10 mg. For other animal species, the corresponding dose as converted per 60 kg weight can be administered.

More specifically, (a) the low molecular weight compound or its salts that inhibit the activity of the protein used in the present invention, (b) the low molecular weight compound or its salts that inhibit the expression of the protein used in the present invention, etc. can be subjected to pharmaceutical manufacturing processes according to publicly known methods and the pharmaceutical composition can be administered. For example, the composition (pharmaceutical composition) for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc. Examples of the composition for parenteral administration are injectable preparations, suppositories, etc. The injectable preparations may include dosage forms such as intravenous, subcutaneous, intracutaneous and intramuscular injections, drip infusions, intraarticular injection, etc. These injectable preparations may be prepared by methods publicly known. For example, the injectable preparations may be prepared by dissolving, suspending or emulsifying the active ingredient described above in a sterile aqueous medium or oily medium conventionally used for injections. As the aqueous medium for injections, there are, for example, physiological saline, an isotonic solution containing glucose and other auxiliary agents, etc., which may be used in combination with an appropriate solubilizing aid such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing aid such as benzyl benzoate, benzyl alcohol, etc. The injection thus prepared is usually filled in an appropriate ampoule. The suppository used for rectal administration may be prepared by blending the aforesaid substance with conventional bases for suppositories.

The antisense polynucleotide described above can be subjected to pharmaceutical manufacturing processes according to publicly known methods and then administered. The antisense polynucleotide alone can be administered directly, or the antisense polynucleotide can be inserted into an appropriate vector such as retrovirus vector, adenovirus vector, adenovirus-associated virus vector, etc., and then administered orally or parenterally to human or a mammal (e.g., rat, rabbit, sheep, swine, bovine, cat, dog, monkey, etc.) in a conventional manner. The antisense polynucleotide may be administered as it stands, or may also be subjected to pharmaceutical manufacturing processes together with a physiologically acceptable carrier such as a pharmaceutical aid to assist its uptake, which are then administered by gene gun or through a catheter such as a catheter with a hydrogel. Alternatively, the antisense polynucleotide may be prepared into an aerosol, which is topically administered into the trachea as an inhaler. Further for the purposes of improving pharmacokinetics, prolonging a half-life and improving intracellular uptake efficiency, the antisense polynucleotide described above is prepared into pharmaceutical preparations (injectable preparations), alone or together with a carrier such as liposome, etc. and the preparations may be administered intravenously, subcutaneously or intraarticularly, or at the site of cancerous lesion, etc. The aforesaid double-stranded RNA or ribozyme, or the aforesaid variant of the protein used in the present invention which acts dominant negatively against the protein used in the present invention or the polynucleotide encoding the same can be likewise prepared into pharmaceutical preparations as in the antisense polynucleotide described above, which preparations can be provided for administration.

The antibody, aptamer, etc. described above can be administered directly as it is or in the form of an appropriate pharmaceutical composition. The pharmaceutical composition used for the administration described above contains the aforesaid antibody or its salt and a pharmacologically acceptable carrier diluent or excipient. Such a composition is provided in the preparation suitable for oral or parenteral administration (e.g., intravenous injection). Preferably, the composition is provided as an inhaler.

Cancers targeted by the P-glycoprotein expression inhibitor or BCRP expression inhibitor of the present invention are not particularly limited, as far as they are cancers to which the aforesaid anticancer agent is applicable.

### (Method for screening the P-glycoprotein expression inhibitor using the activity of inhibiting Ras, Raf, MEK, ERK or RSK as an indicator)

### (Method for screening the BCRP expression inhibitor using the activity of inhibiting Ras, Raf, MEK, ERK or RSK as an indicator)

Next, the present invention provides the method of screening the P-glycoprotein expression inhibitor or the BCRP expression inhibitor using as an indicator the activity of inhibiting MAPK signaling such as the Ras, Raf, MEK, ERK or RSK inhibitory activity.

A preferred embodiment of the screening method of the present invention is a method which comprises evaluating the activity of a test compound for inhibiting MAPK signaling and selecting a compound having the activity of inhibiting MAPK signaling. The "activity of inhibiting MAPK signaling" refers to the activity capable of inhibiting signal transduction mediated by the protein involved in MAPK signaling through inhibition of the expression or activation of the protein involved in MAPK signaling or through destabilization of the protein. Therefore, the activity of inhibiting MAPK signaling can be evaluated not only by the inhibitory activity against MAPK signaling, but also by the activity of inhibiting the expression or activation of the protein involved in MAPK signaling, such as the Ras protein, Raf protein, MEK protein, ERK protein, RSK protein, etc., or by the activity of its destabilization, or the like. The compound thus selected is such a compound that inhibits the expression ofP-glycoprotein or BCRP and can be a candidate compound for the agent for preventing the resistance to anticancer agents.

The activity of inhibiting the expression or activation of the protein involved in MAPK signaling, or the activity of destabilization can be assayed in accordance with methods publicly known. Specifically, the activity of inhibiting MAPK signaling can be evaluated by, e.g., the Ras inhibitory activity, Raf inhibitory activity, MEK inhibitory activity, ERK inhibitory activity, RSK inhibitory activity, or the like. As used herein, the Ras inhibitory activity, Raf inhibitory activity, MEK inhibitory activity, ERK inhibitory activity or RSK inhibitory activity refers to an activity capable of inhibiting signal transduction mediated by the Ras protein, Raf protein, MEK protein, ERK protein or RSK protein through inhibition of the expression or activation of the Ras protein, Raf protein, MEK protein, ERK protein or RSK protein or through inactivation of the protein; or the like.

The Ras inhibitory activity can be determined by the method described in, for example, J. Biol. Chem., 270: 26802-26806 (1995), Cancer Res., 56: 1727-1730 (1996) or Cancer Res., 59: 4919-4926 (1999). The Raf inhibitory activity can be determined by the method described in, for example, Chem. Biol. 6: 559-568 (1999) or Int. J. Clin. Pharmacol. Ther., 40: 567-568 (2002). The MEK inhibitory activity can be determined by the method described in, for example, WO99/01426, J. Immunol., 160, 4175 (1998); J. Biol. Chem., 273, 18623 (1998); J. Biol. Chem., 274, 6168 (1999); J. Biol. Chem., 274, 6747 (1999); Bioorg. Med. Chem. Lett., 8, 2839 (1998), etc. The ERK inhibitory activity can be determined by the method described in, for example, Japanese Laid-Open Patent Publication (Tokkai) No. 2005-330265. The RSK inhibitory activity can be determined by the method described in, for example, Org. Lett., 7: 1097-1099 (2005) or Bioorg. Med. Chem., 14: 3974-3977 (2006).

Examples of the test compound include peptides, proteins, antibodies, non-peptide compounds, synthetic compounds, fermentation products, cell extracts, plant extracts, animal tissue extracts, blood plasma, and the like. These compounds may be novel or publicly known compounds. The test compound may form a salt, and the salts of the compound include physiologically acceptable metal salts, ammonium salts, salts with organic bases, salts with inorganic acids, salts with organic acids, salts with basic or acidic amino acids, etc. Preferred examples of the metal salts include alkali metal salts such as sodium salts, potassium salts, etc.; alkaline earth meal salts such as calcium salts, magnesium salts, barium salts, etc.; aluminum salts, etc. Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Preferred examples of the salts with inorganic acids include salts with hydrochloric acid, hydrobromic acid, nitric acid, sulfuric acid, phosphoric acid, etc. Preferred examples of the salts with organic acids include salts with formic acid, acetic acid, trifluoroacetic acid, .phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of the salts with basic amino acids include salts with arginine, lysine, ornithine, etc., and preferred examples of the salts with acidic amino acids include salts with aspartic acid, glutamic acid, etc.

More specifically, the present invention provides, for example, (1) the method of screening the P-glycoprotein expression inhibitor or BCRP expression inhibitor, which comprises comparing MAPK signaling activities mediated by Ras, Raf, MEK, ERK or RSK of a cell, (i) when a test compound is brought in contact with the cell and (ii) when a test compound is not brought in contact with the cell.

According to this method, first, a test compound is contacted with a cell that expresses the protein involved in MAPK signaling. The "cells" used are cells derived from human or pet or domestic animals including mice, cats, dogs, bovine, sheep, fowl, etc. but are not limited to those of such origins. As the "cell that expresses the protein involved in MAPK signaling," there can be utilized a cell that expresses the protein involved in MAPK signaling or a cell in which an exogenous gene encoding the protein involved in MAPK signaling expresses is transfected and the gene is expressed. The cell in which an exogenous gene encoding the protein involved in MAPK signaling expresses is expressed can be produced generally by transfecting an expression vector wherein the gene encoding the protein involved in MAPK signaling into host cells. The expression vector can be produced by general genetic engineering techniques.

Next, the MAPK signaling activity (e.g., phosphorylation activity) is determined. Specifically, the cells described above are cultured in the cases of (i) and (ii) to assay their MAPK signaling activities. The MAPK signaling activity can be assayed by publicly known methods, for example, through determination of the phosphorylation of Raf, MEK, ERK or RSK by western blotting or ELISA assays using a phosphorylation antibody capable of specifically reacting with these proteins, or through the phosphorylation of transcription factors (CREB, c-Fos, etc.) located downstream of these proteins by western blotting or ELISA using a phosphorylation antibody capable of specifically reacting with these proteins; etc. Also, the MAPK signaling activity can also be determined by assaying the transcription factor located downstream of the protein involved in MAPK signaling, for example, for its ability of transcriptional activation by isolating a promoter region from the target gene encoding the transcription factor in a conventional manner, inserting a labeled gene (e.g., a light-emitting, fluorescent or chromogenic gene such as luciferase, GFP, galactosidase, etc.) downstream of the promoter region and assaying the activity of the labeled gene. The compounds as given above are likewise used as the test compound.

Next, the compound that inhibits (reduces) the MAPK signaling when compared to the case where the test compound is not contacted (control) is selected. A test compound that inhibits (reduces) the MAPK signaling activity in the case of (i) described above, for example, by at least about 20%, preferably at least 30%, more preferably at least about 50% when compared to the case of (ii) described above can be selected as the compound that inhibits (reduces) the MAPK signaling. The compound thus selected is such a compound or its salts that inhibit the expression of P-glycoprotein or BCRP and can be a candidate compound for the agent for preventing the resistance to anticancer agents.

Furthermore, the present invention provides, for example, (2) the method of screening the P-glycoprotein expression inhibitor or the BCRP expression inhibitor, which comprises comparing the expression level of a gene encoding the protein involved in MAPK signaling, such as the Ras protein, Raf protein, MEK protein, ERK protein, RSK protein, etc., of a cell, (i) when a test compound is brought in contact with the cell and (ii) when a test compound is not brought in contact with the cell.

According to this method, first, a test compound is brought in contact with a cell that expresses a gene encoding the protein involved in MAPK signaling as described above. The cells are cells that express a gene encoding the protein involved in MAPK signaling, such as the Ras protein, Raf protein, MEK protein, ERK protein, RSK protein, etc. Preferably used are human vulvar cancer cells A431, human colon cancer cells HCT-15, SW620, human breast cancer cells MCF-7, MDA-MB-231, human non-small lung cancer cells A549, human ovarian cancer cells OVCAR-5, etc.

Next, the expression of a gene encoding the protein involved in MAPK signaling is determined. Specifically, the aforesaid cells are cultured in the cases of (i) and (ii) described above to measure the expression level of a gene encoding the protein involved in MAPK signaling. The gene expression level can be determined by publicly known methods including the measurement of transcription level or translation level, etc. The transcription level of said gene can be determined, for example, by extracting mRNA from cells that express a gene encoding the protein involved in MAPK signaling in a conventional manner and performing northern hybridization or RT-PCR using this mRNA as a template. Alternatively, the transcription level of said gene can also be determined by isolating a promoter region from the gene encoding the protein involved in MAPK signaling in a conventional manner, inserting a labeled gene (which includes, e.g., a detectable gene using light-emission, fluorescence or chromogenicity such as luciferase, GFP, galactosidase, etc. as an indicator but is not limited thereto) downstream of the promoter region and assaying the activity of the labeled gene. The translation level of said gene can be determined by recovering a protein fraction from cells that express a gene encoding the protein involved in MAPK signaling and detecting the expression of the protein involved in MAPK signaling using electrophoresis such as SDS-PAGE, etc. Furthermore, the translation level of the gene can also be determined by performing western blotting using an antibody against a protein involved in MAPK signaling to detect the expression of said protein. The antibody used to detect the protein involved in MAPK signaling is not particularly limited and can be any antibody, so long as it is a detectable antibody; for example, any of a monoclonal antibody and a polyclonal antibody can be used as the antibody. The compounds as given above are likewise used as the test compound.

Next, the compound that inhibits (reduces) the expression level of the gene encoding the protein involved in MAPK signaling when compared to the case where the test compound is not contacted (control) is selected. The compound thus selected is a compound or its salts that inhibit the expression of P-glycoprotein or BCRP and can be a candidate compound for the agent for preventing the resistance to anticancer agents.

The present invention further provides, for example, (3) the method of screening the P-glycoprotein expression inhibitor or the BCRP expression inhibitor, which comprises comparing the activities of the protein involved in MAPK signaling such as the Ras protein, Raf protein, MEK protein, ERK protein, RSK protein, etc., (i) in the presence of a test compound and (ii) in the absence of a test compound. Specifically, the activity of the protein involved in MAPK signaling such as the Ras protein, Raf protein, MEK protein, ERK protein, RSK protein, etc. is assayed in the cases of (i) and (ii) described above. Next, the compound that inhibits (reduces) the activity of the protein involved in MAPK signaling when compared to the case in the absence of the test compound (control) is selected. The activity of the protein involved in MAPK signaling can be assayed by publicly known methods, for example, through determination of the phosphorylation of Raf, MEK, ERK or RSK by western blotting or ELISA assays using a phosphorylation antibody capable of specifically reacting with these proteins, or through determination of the phosphorylation of transcription factors (CREB, c-Fos, etc.) located downstream of these proteins by western blotting or ELISA assays using a phosphorylation antibody capable of specifically reacting with these proteins; etc. Also, the MAPK signaling activity can also be determined by assaying the transcription factor located downstream of the protein involved in MAPK signaling, for example, for the ability of transcriptional activation by isolating a promoter region from the target gene encoding the transcription factor in a conventional manner, inserting a labeled gene (e.g., a light-emitting, fluorescent or chromogenic gene such as luciferase, GFP, galactosidase, etc.) downstream of the promoter region and assaying the activity of the labeled gene. The compound thus selected is a compound or its salt that inhibits the expression of P-glycoprotein expression inhibitor or BCRP expression inhibitor and can be a candidate compound for the agent for preventing the resistance to anticancer agents.

The present invention further provides, for example, (4) the method of screening the P-glycoprotein expression inhibitor, which comprises comparing the stability of the protein involved in MAPK signaling such as the Ras protein, Raf protein, MEK protein, ERK protein, RSK protein, etc., (i) in the presence of a test compound and (ii) in the absence of a test compound. Specifically, the stability of the protein involved in MAPK signaling such as the Ras protein, Raf protein, MEK protein, ERK protein, RSK protein, etc. is assayed in the cases of (i) and (ii) described above. The stability of the protein involved in MAPK signaling can be assayed by publicly known methods, e.g., the method (³⁵S-labelled pulse chase technique) described in Cancer Res., 65: 596-604 (2005). Next, the compound that inhibits (reduces) the stability of the protein involved in MAPK signaling when compared to the case in the absence of the test compound (control) is selected. The compound thus selected is a compound or its salts that inhibit the expression of P-glycoprotein and can be a candidate compound for the agent for preventing the resistance to anticancer agents.

Preferably, the compound selected by the methods described above is further screened by using the expression of P-glycoprotein or BCRP as an indicator. It is preferred that (1) the P-glycoprotein expression inhibitor or BCRP expression inhibitor is screened by comparing the expression level of P-glycoprotein or BCRP in cells, for example, (i) when a test compound is brought in contact with cells and (ii) when a test compound is not brought in contact with cells.

Specifically, the cells described above are cultured in the cases of (i) and (ii) to determine the expression level of a gene encoding P-glycoprotein or BCRP. The gene expression level can be determined by publicly known methods including the measurement of transcription level or translation level, etc. The transcription level of said gene can be determined, for example, by extracting mRNA from cells that express a gene encoding P-glycoprotein or BCRP in a conventional manner and performing northern hybridization or RT-PCR using this mRNA as a template. Alternatively, the transcription level of said gene can also be determined by isolating a promoter region from the gene encoding P-glycoprotein or BCRP in a conventional manner, inserting a labeled gene (which includes, e.g., a detectable gene using light-emission, fluorescence or chromogenicity such as luciferase, GFP, galactosidase, etc. as an indicator but is not limited thereto) downstream of the promoter region and assaying the activity of the labeled gene. Alternatively, the translation level of said gene can be determined by recovering a protein fraction from cells that express a gene encoding P-glycoprotein or BCRP and detecting the expression of P-glycoprotein or BCRP using electrophoresis such as SDS-PAGE, etc. Furthermore, the translation level of the gene can also be determined by performing western blotting using an antibody against P-glycoprotein or BCRP to detect the expression of said protein. The antibody used to detect P-glycoprotein or BCRP is not particularly limited and can be any antibody so long as it is a detectable antibody; for example, any of a monoclonal antibody and a polyclonal antibody can be used as the antibody. The compounds as given above are likewise used as the test compound.

Next, the compound that inhibits (reduces) the expression level of the gene encoding P-glycoprotein or BCRP when compared to the case where the test compound is not contacted (control) is selected. The compound thus selected is a compound or its salts that inhibit the expression of P-glycoprotein or BCRP and can be a candidate compound for the agent for preventing the resistance to anticancer agents.

### (Anticancer agent comprising the P-glycoprotein expression inhibitor which is reduced in resistance acquisition)

### (Anticancer agent comprising the BCRP expression inhibitor which is reduced in resistance acquisition)

The present invention further provides the anticancer agent comprising the P-glycoprotein expression inhibitor which is reduced in resistance acquisition (hereinafter referred to as the "resistance acquisition-reduced anticancer agent"). The resistance acquisition-reduced anticancer agent of the present invention comprises the P-glycoprotein expression inhibitor having both the activity of inhibiting the expression of P-glycoprotein and an anticancer activity. Accordingly, the resistance acquisition-reduced anticancer agent of the present invention can selectively kill cancer cells, inhibit growth of cancer cells and/or induce apoptosis of cancer cells, and at the same time can inhibit the expression of P-glycoprotein thereby to prevent acquisition of drug resistance in cancer cells and is thus useful as the anticancer agent which is reduced in resistance acquisition. The resistance acquisition-reduced anticancer agent of the present invention can comprise the P-glycoprotein expression inhibitor alone, having both the activity of inhibiting the expression of P-glycoprotein and an anticancer activity or can also be formulated with other anticancer drugs.

In addition, the present invention further provides the anticancer agent comprising the BCRP expression inhibitor which is reduced in resistance acquisition (hereinafter referred to as the "resistance acquisition-reduced anticancer agent"). The resistance acquisition-reduced anticancer agent of the present invention comprises the BCRP expression inhibitor having both the activity of inhibiting the expression of BCRP and an anticancer activity. Accordingly, the resistance acquisition-reduced anticancer agent of the present invention can selectively kill cancer cells, inhibit growth of cancer cells and/or induce apoptosis of cancer cells, and at the same time can inhibit the expression of BCRP thereby to prevent acquisition of drug resistance in cancer cells and is thus useful as the anticancer agent which is reduced in resistance acquisition. The resistance acquisition-reduced anticancer agent of the present invention can comprise the BCRP expression inhibitor alone, having both the activity of inhibiting the expression of BCRP and an anticancer activity or can also be formulated with other anticancer drugs.

As the MAPK signaling inhibitor used in the resistance acquisition-reduced anticancer agent of the present invention, the MAPK signaling inhibitor described above can be preferably used; among others, the Ras inhibitor, Raf inhibitor, MEK inhibitor, ERK inhibitor and RSK inhibitor are preferred.

In the case where the P-glycoprotein expression inhibitor or BCRP expression inhibitor used in the present invention is used as the agent described above, the inhibitor can be subjected to pharmaceutical manufacturing processes in a conventional manner.

Examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, , etc. The injectable preparations may include dosage forms such as intravenous, subcutaneous, intracutaneous and intramuscular injections, drip infusions, intraarticular injection, etc. These injectable preparations may be prepared by methods publicly known. The injectable preparations can be prepared, e.g., by dissolving, suspending or emulsifying the P-glycoprotein expression inhibitor described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing aid such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, for example, sesame oil, soybean oil, etc., which may be used in combination with a solubilizing aid such as benzyl benzoate, benzyl alcohol, etc. The injection thus prepared is preferably filled in an appropriate ampoule. The suppository used for rectal administration may be prepared by blending the aforesaid the P-glycoprotein expression inhibitor with conventional bases for suppositories.

Advantageously, the pharmaceutical compositions for oral or parenteral use described above are prepared into pharmaceutical preparations in a unit dose suitable for the dose of active ingredient. Such unit dose preparations include, for example, tablets, pills, capsules, injections (ampoules), suppositories, etc. The amount of the aforesaid P-glycoprotein expression inhibitor or BCRP expression inhibitor contained is generally about 5 to 500 mg per dosage unit form; especially in the form of injection, it is preferred that the aforesaid P-glycoprotein expression inhibitor or BCRP expression inhibitor is contained in about 5 to 100 mg and in about 10 to 250 mg for the other forms.

Each of the compositions described above may further contain other active ingredients unless formulation causes any adverse interaction with the compound described above.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the P-glycoprotein expression inhibitor or BCRP expression inhibitor described above may vary depending upon its effect, target disease, subject to be administered, conditions, route of administration, etc. For example, when the P-glycoprotein expression inhibitor used in the present invention is orally administered for the purpose of treating, for example, lung cancer, the aforesaid agent is generally administered to an adult (as 60 kg body weight) in a daily dose of about 0.1 to about 100 mg, preferably about 1.0 to about 50 mg and more preferably about 1.0 to about 20 mg. In parenteral administration, a single dose of the aforesaid agent may vary depending upon target disease, subject to be administered, conditions, route of administration, etc. When the P-glycoprotein expression inhibitor used in the present invention is administered to an adult (as 60 kg body weight) in the form of an injectable preparation for the purpose of treating, for example, lung cancer (e.g., myocardial infarction, unstable angina, etc.), it is advantageous to administer the said agent by way of injection in a daily dose of about 0.01 to about 30 mg, preferably about 0.1 to about 20 mg, and more preferably about 0.1 to about 10 mg. For other animal species, the corresponding dose as converted per 60 kg weight can be administered.

The P-glycoprotein expression inhibitor or BCRP expression inhibitor of the present invention can also be used in combination with hormonal therapeutic agents, anticancer drugs (e.g., chemotherapeutic agents, immunotherapeutic agents, tyrosine kinase signaling inhibitors (drugs inhibiting the actions of cell growth factors and their receptors), etc. (hereinafter briefly referred to as "concomitant drugs").

The compound of the present invention alone exhibits excellent anticancer effects even when it is as a single drug but its effects can be more potentiated in combined administration with one or more of the concomitant drugs described above (polypharmacy).

Also, the P-glycoprotein expression inhibitor of the present invention can be used as the agent for preventing the resistance to an anticancer agent and is extremely useful as a drug for treating cancers with acquired resistance when the P-glycoprotein expression inhibitor is used in combination with anticancer drugs (e.g., hormonal therapeutic agents, chemotherapeutic agents, immunotherapeutic agents, or drugs inhibiting the actions of cell growth factors and their receptors).

More specifically, concomitant use of (A) the P-glycoprotein expression inhibitor of the present invention and (B) the aforesaid anticancer drugs to which cancer cells might acquire resistance restores the therapeutic efficacy against cancers with acquired resistance, and therefore, the composition or concomitant drug comprising these ingredients (A) and (B) is useful as a new drug for the treatment of cancers.

Also, concomitant use of (A) the P-glycoprotein expression inhibitor of the present invention and (B) the aforesaid anticancer drugs to which cancer cells might acquire resistance can prevent cancer cells from acquiring resistance to provide medical treatment of cancers. Accordingly, the composition or concomitant drug comprising these ingredients (A) and (B) is useful as a new drug for the treatment of cancers.

Furthermore, the BCRP expression inhibitor of the present invention can be used as the agent for preventing the resistance to an anticancer agent and is extremely useful as a drug for treating cancers with acquired resistance when the BCRP expression inhibitor is used in combination with anticancer drugs (e.g., hormonal therapeutic agents, chemotherapeutic agents, immunotherapeutic agents, or drugs inhibiting the actions of cell growth factors and their receptors).

More specifically, concomitant use of (A) the BCRP expression inhibitor of the present invention and (B) the aforesaid anticancer drugs to which cancer cells might acquire resistance restores the therapeutic efficacy against cancers with acquired resistance, and therefore, the composition or concomitant drug comprising these ingredients (A) and (B) is useful as a new drug for the treatment of cancers.

Also, concomitant use of BCRP (A) of the present invention and the aforesaid anticancer drugs (B) to which cancer cells might acquire resistance can prevent cancer cells from acquiring resistance to provide medical treatment of cancers. Accordingly, the composition or concomitant drug comprising these ingredients (A) and (B) is useful as a new drug for the treatment of cancers.

Such anticancer drugs to which cancer cells might acquire resistance are not limited as far as they are anticancer drugs with P-glycoprotein-induced or BCRP-induced resistance and include, for example, anthracyclines such as doxorubicin hydrochloride, daunomycin, epirubicin hydrochloride, adriamycin, etc.; vinca alkaloids such as vincristine, etc.; taxanes such as paclitaxel, docetaxel, etc.; and other drugs including hormonal therapeutic agents, chemotherapeutic agents, immunotherapeutic agents, or drugs inhibiting the actions of cell growth factors and their receptors; and the like.

Examples of "hormonal therapeutic agents" include fosfestrol, diethylstylbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogens (e.g., tamoxifen citrate, toremifene citrate, etc.), ER downregulators (e.g., fulvestrant, etc.), human menopausal gonadotropin, follicle stimulating hormone, pill dosage forms, mepitiostane, testrolactone, aminoglutethimide, LH-RH agonists (e.g., goserelin acetate, buserelin, leuprorelin, etc.), droloxifene, epitiostanol, ethinylestradiol sulfonate, aromatase inhibitors (e.g., fadrozole hydrochloride, anastrozole, retrozole, exemestane, vorozole, formestane, etc.), anti-androgens (e.g., flutamide, bicartamide, etc.), 5α-reductase inhibitors (e.g., finasteride, dutasteride, epristeride, etc.), adrenocorticohormone drugs (e.g., dexamethasone, prednisolone, betamethasone, triamcinolone, etc.), androgen synthesis inhibitors (e.g., abiraterone, etc.), retinoid and drugs that retard retinoid metabolism (e.g., liarozole, etc.), and the like; among others, LH-RH agonists (e.g., goserelin acetate, buserelin, leuprorelin, etc.) are preferable.

The "chemotherapeutic agents" include, for example, alkylating agents, antimetabolites, anticancer antibiotics, anticancer agents derived from plants, etc.

Examples of "alkylating agents" include nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, estramustine sodium phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin, etc.

Examples of "antimetabolites" include mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emmitefur, etc.), aminopterin, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, ambamustine, etc.

Examples of "anticancer antibiotics" include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, etc.

Examples of "anticancer agents derived from plants" include etoposide, etoposide phosphate, vinblastine sulfate, vincristine sulfate, vindesine sulfate, teniposide, paclitaxel, docetaxel, vinorelbine, etc.

Examples of "immunotherapeutic agents (BRM)" include picibanil, krestin, sizofiran, lentinan, ubenimex, interferons, interleukins, macrophage colony-stimulating factor, granulocyte colony-stimulating factor, erythropoietin, lymphotoxin, BCG vaccine, Corynebacterium parvum, levamisole, polysaccharide K, procodazole, etc.

The "cell growth factors" in the "drugs inhibiting the actions of cell growth factors and their receptors" can be any substance so long as they are materials which stimulate the cell growth and normally, peptides which have a molecular weight of 20,000 or less and bind to their receptors to exhibit the actions in a lower level can be used as the factors. Specific examples are (1) EGF (epidermal growth factor) or substances having substantially the same activity as EGF [e.g., EGF, hereglin, etc.], (2) insulin or substances having substantially the same activity as insulin [e.g., insulin, IGF (insulin-like growth factor)-1, IGF-2, etc.], (3) FGF (fibroblast growth factor) or substances having substantially the same activity as FGF [e.g., acidic FGF, basic FGF, KGF (keratinocyte growth factor), FGF-10, etc.], (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), etc.] and the like.

The "receptors of the cell growth factors" can be any receptor as long as they are capable of binding to the cell growth factors described above, and specific examples are EGF receptor, hereglin receptor (HER2), insulin receptor, IGF receptor, FGF receptor-1 or FGF receptor-2, etc.

Examples of "drugs inhibiting the actions of cell growth factors" include trastuzumab (Herceptin (trademark); HER2 antibody), imatinib mesylate, ZD1839 or, an antibody against VEGF (e.g., bevacizumab), an antibody against VEGF receptor, gefitinib, erlotinib, etc.

In addition to the aforesaid agents/drugs, there can be also used L-asparginase, aceglatone, procarbazine hydrochloride, protoporphyrin-cobalt complex, mercury-hematoporphyrin sodium, topoisomerase I inhibitors (e.g., irinotecan, topotecan, etc.), topoisomerase II inhibitors (e.g., sobzoxan, etc.), differentiation-inducing agent (e.g., retinoid, vitamin D group, etc.), angiogenesis inhibitors (e.g., thalidomide, SU11248, etc.), α-blockers (e.g., tamsulosin hydrochloride, naftopidil, urapidil, alfuzosin, terazosin, prazosin, silodosin, etc.), serine-threonine kinase inhibitors, endothelin receptor antagonists (e.g., atrasentan, etc.), proteasome inhibitors (e.g., bortezomib, etc.), Hsp90 inhibitors (e.g., 17-AAG, DMAG (17-desmethoxy-17-N,N-dimethylaminoethylamino-geldanamycin), etc.), spironolactones, minoxidil, 11α-hydroxyprogesterone, bone resorption inhibitors/bone metastasis suppressors (e.g., zoledronic acid, alendronic acid, pamidronic acid, etidronic acid, ibandronic acid, clodronic acid), etc.

The novel anticancer agent, agent for preventing the resistance to anticancer agents and cancer-treating agent of the present invention may be administered concomitantly with conventional pharmaceutical preparations as they are, in which these ingredients are conventionally used; alternatively, they may also be prepared into a new pharmaceutical composition containing these ingredients. The dosage form of these pharmaceutical compositions includes oral preparations, injectable preparations (including intramuscular, subcutaneous and intravenous injections), suppositories, topical preparations (patch, liniment), etc.

The aforesaid agents/drugs including the cancer-treating agent, etc. of the present invention may be in such a dosage form that the P-glycoprotein expression inhibitor used in the present invention and the anticancer agent used in the present invention can exhibit their actions simultaneously. For example, the P-glycoprotein expression inhibitor and the anticancer agents used in the present invention may be formulated together into one pharmaceutical composition (e.g., tablets (including dragees and film-coated tablets), pills, granules, powdery preparations, capsules (including soft capsules), syrup, emulsions, suspensions, injections, suppositories, etc.) to form a combination preparation. Also, the agents/drugs including the cancer-treating agent, etc. of the present invention may be in the form of a kit comprising the P-glycoprotein expression inhibitor used in the present invention and the anticancer agent used the present invention. In this case, the P-glycoprotein expression inhibitor used in the present invention and the anticancer agent used the present invention may be administered with time difference, as far as the P-glycoprotein expression inhibitor used in the present invention and the anticancer agent used the present invention can act simultaneously, but concomitant administration is preferred.

The ratio in dose (formulation ratio) of the P-glycoprotein expression inhibitor to the anticancer agent in the agents/drugs including the cancer-treating agent, etc. of the present invention varies depending upon kind and/or combination of the P-glycoprotein expression inhibitor and anticancer agent used in the present invention, subject to be administered, target disease, conditions, route of administration, etc., but the ratio is, for example, approximately 1:500 to 500:1, preferably approximately 1:100 to 100:1, more preferably 1:10 to 10:1, and particularly preferably 1:5 to 5:1. Also, the ratio in dose (formulation ratio) of the BCRP expression inhibitor to the anticancer agent in the agents/drugs including the cancer-treating agent, etc. of the present invention varies depending upon kind and/or combination of the P-glycoprotein expression inhibitor and anticancer agent used in the present invention, subject to be administered, target disease, conditions, route of administration, etc., but the ratio is, for example, approximately 1:500 to 500:1, preferably approximately 1:100 to 100:1, more preferably 1:10 to 10:1, and particularly preferably 1: 5 to 5:1.

Where the P-glycoprotein expression inhibitor and anticancer agent used in the present invention are employed in the form of the composition described above, the inhibitor and the anticancer agent can be subjected to pharmaceutical manufacturing processes in a conventional manner, e.g., by the methods described above. Also, where the BCRP expression inhibitor and the anticancer agent are used as the agent described above, the BCRP expression inhibitor used in the present invention are employed in the form of the composition described above, the inhibitor and the anticancer agent can be subjected to pharmaceutical manufacturing processes in a conventional manner, e.g., by the methods described above, etc.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injections, etc. These injectable preparations may be prepared by methods publicly known. For example, the injectable preparations may be prepared by dissolving, suspending or emulsifying the agent described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing aid such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing aid such as benzyl benzoate, benzyl alcohol, etc. The injection thus prepared is usually filled in an appropriate ampoule. The suppository used for rectal administration may be prepared by blending the aforesaid agent with conventional bases for suppositories.

Advantageously, the oral or parenteral pharmaceutical compositions described above are prepared into pharmaceutical preparations with a unit dose suitable for a dose of the active ingredients. Such unit dose preparations include, for example, tablets, pills, capsules, injections (ampoules), suppositories, etc. The amount of each ingredient contained is generally 5 to 500 mg per dosage unit form; it is preferred that the P-glycoprotein expression inhibitor and the anticancer agent are contained, respectively, in about 5 to about 100 mg especially in the form of injection, and in 10 to 250 mg for the other forms.

Each of the compositions described above may further contain other active ingredients unless the formulation causes any adverse interaction with the materials described above.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the above agent may vary depending upon its action, target disease, subject to be administered, conditions, route of administration, etc. For example, when the P-glycoprotein expression inhibitor and anticancer agent used in the present invention are orally administered for the purpose of treating, for example, lung cancer, the aforesaid agent is generally administered to an adult (as 60 kg body weight) in a daily dose of about 0.1 to about 100 mg, preferably about 1.0 to about 50 mg and more preferably about 1.0 to about 20 mg. In parenteral administration, the dose of the aforesaid agent may vary depending upon target disease, subject to be administered, conditions, route of administration, etc. When the P-glycoprotein expression inhibitor and anticancer agent used in the present invention are administered to an adult (as 60 kg body weight) in the form of an injectable preparation for the purpose of treating, for example, lung cancer, it is advantageous to administer the aforesaid agent by way of intravenous injection in a daily dose of about 0.01 to about 30 mg, preferably about 0.1 to about 20 mg, and more preferably about 0.1 to about 10 mg. For other animal species, the corresponding dose as converted per 60 kg weight can be administered.

In the specification and sequence listings, where nucleotides, amino acids, etc. are denoted by their abbreviations, they are based on conventional codes in accordance with the IUPAC-IUB Commission on Biochemical Nomenclature or by the common codes in the art, examples of which are shown below. For amino acids that may have the optical isomer, L form is presented unless otherwise indicated.

### EXAMPLES

Next, the present invention will be described below in detail with reference to EXAMPLES but is not deemed to be limited to these EXAMPLES.

### PREPARATION EXAMPLE 1 (Preparation of the strain expressing P-glycoprotein at a high level)

### (1) MDR1 gene

In the present invention, the gene called human wild-type MDR1 cDNA isolated from a human adrenal cDNA library was used as human wild-type MDR1 cDNA (see Biochem. Biophys. Res. Commun., 162: 224-231 (1989)).

### (2) MDR1expression plasmid

Wild-type MDR1-expressing retrovirus vector plasmid pHaMDR used in the present invention is the plasmid described in Nature Biotechnology, 12: 694-698 (1994).

### (3) Preparation of MDR1 retrovirus

The retrovirus liquid of wild-type MDR1-expressing retrovirus HaMDR used in the present invention was prepared as follows. After calcium phosphate transfection was performed to introduce a pHaMDR plasmid to PA317 cells, which constitute a mouse amphotropic retrovirus packaging cell line, 35 ng/ml of vincristine was used for selecting vincristine-resistant cells. The thus selected vincristine-resistant cells were subjected to cloning by limiting dilution, and the culture supernatant of retrovirus-producing cells 3P26 was collected. The 3P26 cells are described in Clin. Cancer Res., 3: 947-954 (1997).

The culture supernatant of 3P26 cells was collected and filtered through a 0.45 micrometer filter to give the retrovirus liquid.

### (4) Preparation of MCF-7/MDR1 cells and MDA-MB-231/MDR cells

The HaMDR retrovirus liquid was added to a culture of human breast cancer cells MCF-7 to perform gene transfer. Gene transferred cells were selected from the retrovirus-added cells using 6 ng/ml of vincristine. The selected cells were named MCF-7/MDR1. MDA-MB-231/MDR was prepared in a manner similar to the MCF-7/MDR cells.

### (5) Preparation of SW620-14 cells

Colon cancer cell line SW620 cells were prepared by using the limiting dilution method. Specifically, colon cancer cell line SW620 cells were plated at one cell per well in a 96-well plate (manufactured by IWAKI GLASS Co. Ltd.). The cells only in the wells where the cells were grown were transferred to a 24-well plate (manufactured by IWAKI GLASS Co. Ltd.). After the cells were grown, the cells were further transferred to a 100 mm-dish (manufactured by IWAKI GLASS Co. Ltd.) for culture. In the clones thus obtained, clones which expressed P-glycoprotein at a high level were confirmed by FACS to give clone 14 carrying P-glycoprotein expressed at a high level. This cell was named SW620-14 cell. FACS was performed by the procedure described in page 14, line 12 et seq.

### EXAMPLE 1 (Screening of endogenous P-glycoprotein expression inhibitor)

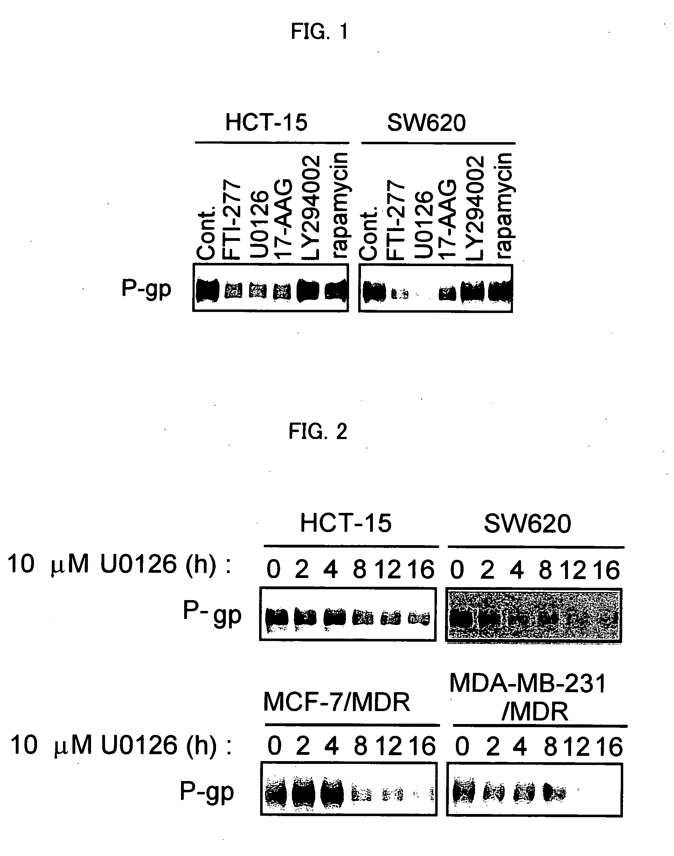

In Escherichia coli-derived HCT-15 cells (obtained from Developmental Therapeutics Program, National Cancer Institute, National Institutes of Health, Bethesda, MD, USA) and Escherichia coli-derived SW620 cells (obtained from Developmental Therapeutics Program, National Cancer Institute, National Institutes of Health, Bethesda, MD, USA), which expressed P-glycoprotein at a high level, screening of drugs that reduced the expression level of P-glycoprotein was performed by western blotting. After Ras inhibitor (farnesyltransferase inhibitor) FTI-277 (manufactured by EMD Bioscience), MEK inhibitor U0126 (manufactured by Cell Signaling Technology), Hsp90 inhibitor 17-AAG (17-(allylamino)-17-demethoxygeldanamycin; purchased from Almone Labs.) PI3K inhibitor LY294002 (2-(4-morpholinyl)-8-phenyl-1(4H)-benzopyran-4-one; manufactured by Merck Calbiochem) or mTOR inhibitor rapamycin (23,27-epoxy-3H-pyrido[2,1-c][1,4]oxaazacyclohentriacontine; manufactured by Sigma) were added, respectively, to DMEM medium supplemented with 7% fetal calf serum, HCT-15 cells or SW620 cells were incubated for 12 hours. The cells were incubated at the initial cell numbers of 300,000/6 cm Petri dish for HCT-15 and 500,000/6 cm Petri dish for SW620, respectively, in carbon dioxide concentration of 5% at a temperature of 37°C. After incubation for 12 hours, each drug was added to the cells followed by further incubation. Then, the P-glycoprotein expression level was assessed by western blotting using an anti-P-glycoprotein antibody (manufactured by Zymed Laboratories Inc., trade name: Multidrug Resistance 1+3 (MDR, P-glycoprotein) (Host: Mouse, Clone: C219)). The cells incubated without adding any drug were used for control (7% fetal calf serum-supplemented DMEM medium added with no drug). The protein of 10 µg each was electrophoresed in each lane. The results are shown in FIG 1. The protein was quantified by Bradford assay (Bio-Rad protein assay dye reagent). More specifically, the incubated cells were recovered with a cell scraper and centrifuged (5,000 rpm x 3 mins.) to pellet down. A lysis buffer containing 0.2% NP-40 was added to the cell pellets. While vortexing every 5 minutes, the cell membrane and cytoplasmic fractions were solubilized on ice for 30 minutes in total. Using the supernatant after centrifugation (15,000 rpm x 15 mins.) as a sample, the protein was quantified.

In the presence of Ras inhibitor (farnesyltransferase inhibitor) FTI-277 or MEK inhibitor U0126, the P-glycoprotein expression level was down-regulated in HCT-15 cells and SW620 cells to 20% of control or less, whereas in the presence of PI3K inhibitor LY294002 or mTOR inhibitor rapamycin, the expression of P-glycoprotein was not down-regulated either in HCT-15 cells or SW620 cells (FIG 1).

The expression level of endogenously expressed P-glycoprotein was markedly reduced by adding the Ras inhibitor or MEK inhibitor. It was observed that the Ras inhibitor and MEK inhibitor had the effect of inhibiting the expression of P-glycoprotein.

### EXAMPLE 2 (Inhibition of P-glycoprotein expression by MEK inhibitor U0126 with passage of time)

Inhibitory effects of MEK inhibitor U0126 on the expression level of P-glycoprotein in HCT-15 and SW620 cells prepared by the same procedure as described in PREPARATION EXAMPLE 1 in which endogenous P-glycoprotein was expressed and in the MCF-7/MDR and MDA-MB-231/MDR cells in which exogenous P-glycoprotein was expressed were monitored with passage of time in a manner similar to EXAMPLE 1.

Specifically, MEK inhibitor U0126 was added to 7% fetal calf serum-supplemented DMEM medium in a final concentration of 10 µM, followed by incubation for 0 to 16 hours. The P-glycoprotein expression level was then confirmed by western blotting. The results are shown in FIG 2.

In the presence of MEK inhibitor U0126, the expression level of P-glycoprotein in HCT-15 and SW620 cells was reduced to 10 to 20% of control in 4 to 8 hours later. Similarly in the presence of U0126, the expression level of P-glycoprotein in MCF-7/MDR and MDA-MB-231/MDR cells was reduced to 10% of control or less in 8 to 12 hours. Any change was not observed in the expression level of MDR1 mRNA.

When MEK inhibitor U0126 was added to the HCT-15 and SW620 cells in which endogenous P-glycoprotein was expressed, the expression level of P-glycoprotein was reduced with passage of time. It was observed that the expression level of P-glycoprotein was markedly reduced in a short period of 4 to 8 hours. Also when MEK inhibitor U0126 was added to the MCF-7/MDR and MDA-MB-231/MDR cells in which MDR1 gene as a P-glycoprotein gene was introduced to express exogenous P-glycoprotein, the expression level of P-glycoprotein was reduced with passage of time. It was observed that the expression level of P-glycoprotein was markedly reduced in 8 to 12 hours later.

MEK inhibitor U0126 showed the effect of inhibiting P-glycoprotein in the shortest period of time as compared to P-glycoprotein expression inhibitors known so far.

### EXAMPLE 3 (Inhibition of P-glycoprotein expression by MEK inhibitor PD098059 with passage of time)

Using MEK inhibitor PD098059 (purchased from Cell Signaling Technology; see English, J. et al. (1999) Exp. Cell Res. 253, 255), which has an action mechanism different from U0126, inhibitory effects of MEK inhibitor PD098059 on the expression level of P-glycoprotein in the HCT-15 and SW620 cells in which endogenous P-glycoprotein was expressed and in the MCF-7/MDR and MDA-MB-231/MDR cells in which exogenous P-glycoprotein was expressed were monitored with passage of time in a manner similar to EXAMPLE 1.

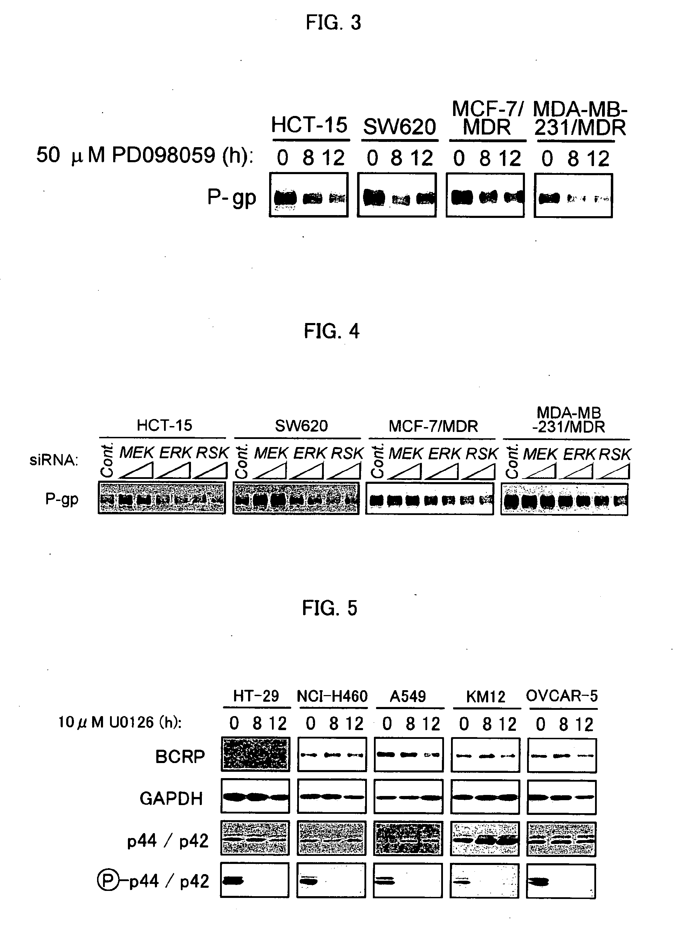

Specifically, MEK inhibitor PD098059 was added to 7% fetal calf serum-supplemented DMEM medium in a final concentration of 50 µM, followed by incubation for 8 hours or 12 hours. The P-glycoprotein expression level was then confirmed by western blotting. The results are shown in FIG 3.

When the cells were incubated in the presence of MEK inhibitor PD098059, the expression level of P-glycoprotein was reduced in any cells to 10 to 20% of control in 8 or 12 hours later.

It was observed that the expression level of P-glycoprotein was reduced in the same time period also in the presence of MEK inhibitor PD098059 having an action mechanism different from that of MEK inhibitor U0126. Accordingly, these drugs are useful as potent P-glycoprotein expression inhibitors. These MAPK signaling inhibitors can reduce the expression of P-glycoprotein in an extremely short period of time to overcome anticancer drug resistance induced by P-glycoprotein.

### EXAMPLE 4 (Inhibition ofP-glycoprotein expression mediated by MAPK signaling gene siRNA)

Using siRNAs of MAPK signaling genes MEK1 and 2, ERK1 and 2 and RSK1, 2 and 3, the effect of inhibiting P-glycoprotein expression by knockdown of MAPK signaling gene was monitored by western blotting. As cells there were used the HCT-15 and SW620 cells in which endogenous P-glycoprotein was expressed and the MCF-7/MDR and MDA-MB-231/MDR cells in which exogenous P-glycoprotein was expressed. These cells were obtained in the same manner as in EXAMPLE 1 or 2.

HCT-15 cells and MCF-7/MDR cells were plated in DMEM medium supplemented with 7% fetal calf serum at 200,000 cells per 6 cm dish (uncoated culture dish, manufactured by Asahi Techno Glass Corp.) and the SW620 and MDA-MB-231/MDR cells at 300,000 cells per 6 cm dish. After incubation overnight (about 16 hours), Control siRNA (QIAGEN; trade name: Control (non-silencing) siRNA, Model: 1022076) or MEK1 siRNA (QIAGEN; trade name: Hs_MAP2K1_6_HP Validated siRNA, Model: S100300699), MEK2 siRNA (QIAGEN; trade name: Hs_MAP2K2_5_HP Validated siRNA, Model: S102225090), ERK1 siRNA (Cell Signaling Technology; trade name: SignalSilence p44 MAPK siRNA (Human Specific), Model: 6436), ERK2 siRNA (Cell Signaling Technology; trade name: SignalSilence Pool p42 MAPK siRNA (Human Specific), Model: 6391), RSK1 siRNA (QIAGEN; trade name: Hs_RPS6KAl_10_HP Validated siRNA, Model: S102223067), RSK2 siRNA(QIAGEN; trade name: Hs_RPS6KA2_9_HP Validated siRNA, Model: S102224999), RSK3 siRNA (QIAGEN; trade name: Hs_RPS6KA3_6_HP Validated siRNA, Model: S100288197) were prepared in a final concentration of 25 nM or 50 nM, respectively, and cationic lipids for gene delivery (trade name "Lipofectamine (trademark) 2000 Reagent" manufactured by Invitrogen) were blended according to the procedure described in the instruction manual. The mixture was added to the cells, respectively. Following the addition, the cells were further incubated at 37°C for 48 hours in a 5% carbon dioxide incubator, and the P-glycoprotein expression level was assayed by western blotting. The results are shown in FIG 4. The amounts of siRNA added to the cells corresponding to the respective lanes in FIG 4 are as follows. That is, in the first lane (Cont.) from the left, Control siRNA was added in a final concentration of 50 nM; in the second lane (MEK, left), MEK1 siRNA at a final concentration of 12.5 nM was mixed with MEK2 siRNA at a final concentration of 12.5 nM and the mixture was added (25 nM as the final concentration of siRNA); in the third lane (MEK, right), MEK1 siRNA at a final concentration of 25 nM was mixed with MEK2 siRNA at a final concentration of 25 nM and the mixture was added (50 nM as the final concentration of siRNA); in the fourth lane (ERK, left), ERK1 siRNA at a final concentration of 12.5 nM was mixed with ERK2 siRNA at a final concentration of 12.5 nM and the mixture was added (25 nM as the final concentration of siRNA); in the fifth lane (ERK, right), ERK1 siRNA at a final concentration of 25 nM was mixed with ERK2 siRNA at a final concentration of 25 nM and the mixture was added (50 nM as the final concentration of siRNA); in the sixth lane (RSK, left), RSK1 siRNA at a final concentration of 8.3 nM was mixed with RSK2 siRNA at a final concentration of 8.3 nM and RSK3 siRNA at a final concentration of 8.3 nM, and the mixture was added (25 nM as the final concentration of siRNA); and in the seventh lane (RSK, right), RSK1 siRNA at a final concentration of 16.7 nM was mixed with RSK2 siRNA at a final concentration of 16.7 nM and RSK3 siRNA at a final concentration of 16.7 nM, and the mixture was added (25 nM as the final concentration of siRNA).

In any cases no significant difference noted in the P-glycoprotein expression levels of the MEK1/2 siRNA-transfected cells as compared with the control, whereas in the ERK1/2 siRNA-transfected cells, the P-glycoprotein expression levels decreased dose-dependently to approximately 50% as compared with the control. Also, the P-glycoprotein expression levels in the RSK1/2/3 siRNA-transfected cells decreased dose-dependently to 10 to 20%, as compared with the control. These results indicate that MAPK signaling siRNAs, especially RSK1 siRNA inhibited the expression of P-glycoprotein extremely efficiently.

Inhibitory effects ofMEK inhibitor U0126 on the BCRP expression level in human colon cancer HT-29 and KM12 cells, human non-small-cell lung cancer NCI-H460 and A549 cells and human ovarian cancer OVACAR-5 cells, in which endogenous BCRP was expressed were monitored with passage of time.

Inhibitory effects ofMEK inhibitor U0126 on the BCRP expression level in human colon cancer-derived HT-29 and KM12 cells (each obtained from Developmental Therapeutics Program, National Cancer Institute, National Institute of Health, Bethesda, MD, USA), human non-small-cell lung cancer NCI-H460 and A549 cells (each obtained from Developmental Therapeutics Program, National Cancer Institute, National Institute of Health, Bethesda, MD, USA) and human ovarian cancer OVACAR-5 cells (obtained from Developmental Therapeutics Program, National Cancer Institute, National Institute of Health, Bethesda, MD, USA), in which BCRP was expressed at a high level, were monitored with passage of time.

Specifically, MEK inhibitor U0126 (manufactured by Cell Signaling Technology) was added to 7% fetal calf serum-supplemented DMEM medium in a final concentration of 10 µM, followed by incubation. The cells were incubated at the initial cell numbers of 200,000/6 cm Petri dish for HT-29, 200,000/6 cm Petri dish for KM12, 200,000/6 cm Petri dish for NCI-H460, 200,000/6 cm Petri dish for A549, and 200,000/6 cm Petri dish for OVACAR-5, respectively, in carbon dioxide concentration of 5% at a temperature of 37°C. After incubation for 8 hours or 12 hours, each drug was added to the cells followed by further incubation. Thereafter, the BCRP expression level was assessed by western blotting using an anti-BCRP antibody (manufactured by Chemicon International Inc., trade name: BXP-21). The protein of 10 µg each was electrophoresed in each lane. The results are shown in FIG 5.

The protein was quantified by Bradford assay (Bio-Rad protein assay dye reagent). More specifically, the incubated cells were recovered with a cell scraper and centrifuged (5,000 rpm x 3 mins.) to pellet down. A lysis buffer containing 0.2% NP-40 was added to the cell pellets. While vortexing every 5 minutes, the cell membrane and cytoplasmic fractions were solubilized on ice for 30 minutes in total. Using the supernatant after centrifugation (15,000 rpm x 15 mins.) as a sample, the protein was quantified.

The effects ofMEK inhibitor U0126 were assessed using the phosphorylated form of p44/p42ERK as an indicator. The results are shown in FIG 5. In FIG 5, the expression levels of GAPDH (glyceraldehyde-3-phosphate dehydrogenase) are also shown.

In the presence of MEK inhibitor U0126, the expression level of BCRP was reduced in human colon cancer cells, human non-small-cell lung cancer cells and human ovarian cancer cells in 12 hours later.

It was demonstrated that the expression of BCRP was suppressed by inhibiting the MAPK signaling system using the MEK inhibitor.

### EXAMPLE 5 (Increased sensitivity to paclitaxel)

Colon cancer cells HCT-15 (1 x 10⁵/60 mm-dish) in which endogenous P-glycoprotein was expressed, SW620-14 (2 x 10⁵/60 mm-dish) obtained in PREPARATION EXAMPLE 1, breast cancer cells MCF-7/MDR (1 x 10⁵/60 nim-dish) and MDA-MB-231/MDR (2 x 10⁵/60 mm-dish) in which exogenous P-glycoprotein was expressed were incubated for 16 hours. Then, 10 µmol/L U0126 (manufactured by Cell Signaling Technology) was added to the cells. While exchanging U0126-containing medium every 24 hours, incubation was continued for 72 hours in total. These cells were obtained as in EXAMPLE 1 or 2, unless otherwise indicated.

The IC₅₀ concentration (50% growth inhibitory concentration) of paclitaxel was previously determined for each cell, namely, 100 nM of paclitaxel against the HCT-15 cells, 5 nM against the SW620-14 cells, 4 nM against the MCF-7/MDR cells and 800 nM against MDA-MB-231/MDR cells; paclitaxel was added to the cells together with 10 µmol/L U0126 in 0-, 1- and 3-fold concentrations, respectively, followed by further incubation for 24 hours. In the control group, the cells were incubated in U0126-free medium for 72 hours in a similar manner and then paclitaxel alone was added thereto followed by incubation for 24 hours. Specifically, the IC₅₀ concentration (50% growth inhibitory concentration) for paclitaxel was determined by the cell growth inhibition tests. The cells were plated in 12-well plates (manufactured by IWAKI GLASS Co. Ltd.), respectively, at 5,000 cells/ml/well. Subsequently, the drug diluted in the medium to different concentrations was added in a volume of 1 ml per well. The plates were placed in a 5% carbon dioxide gas incubator and incubation was performed at 37°C for 5 days. Five days after, the cells were rinsed with phosphate buffer and detached with 0.5 ml of trypsin-EDTA. The cells were then suspended in 1 ml of the medium. The cell suspension in each well was added into a beaker containing 9 ml of CELLPACK diluent (TOA Medical Electronics Co., Ltd.), and the number of cells was counted with a Sysmex CDA-500 automated cell counter (TOA Medical Electronics Co., Ltd.). As a result, the paclitaxel concentration (IC₅₀) causing 50% inhibition in cell number was determined.

After recovery of the cells, the cell membrane and cytoplasmic fractions were solubilized. Changes in P-glycoprotein, production of cleaved PARP (poly (ADP-ribose) polymerase) fragments and GAPDH were confirmed by western blotting. Antibodies used for detecting the respective proteins in the western blotting analysis were an anti-Multidrug Resistance 1+3 monoclonal antibody (C219) (manufactured by Zymed Laboratories Inc.) against P-glycoprotein, an anti-PARP p85 fragment polyclonal antibody (manufactured by Promega Corp.) against PARP, and an anti-glyceraldehyde-3-phosphate dehydrogenase monoclonal antibody (manufactured by Chemicon International Inc.) against GAPDH, respectively.

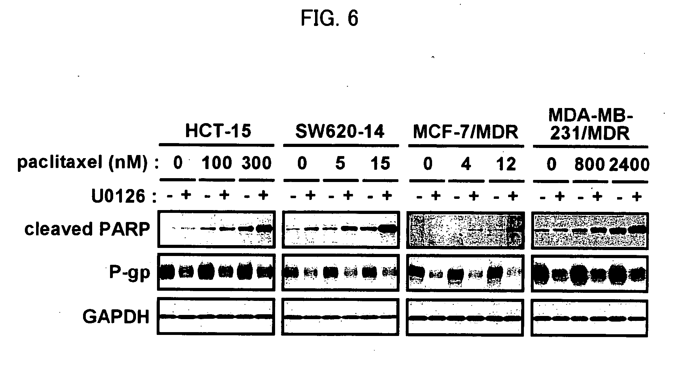

PARP is a substrate of caspase-3, which is an effector of apoptosis. When the apoptosis signal is amplified by anticancer drugs or the like, caspase-3 is activated to cleave PARP. In this experiment, the appearance of cleaved PARP fragment was confirmed as an indicator of apoptosis signaling. The results are shown in FIG 6.

As shown in FIG 6, it was confirmed that the sensitivity to paclitaxel in the cancer cells was increased by the MEK inhibitor.

### EXAMPLE 6 (Increased uptake of fluorogenic substrate Rhodamine 123 for P-glycoprotein)

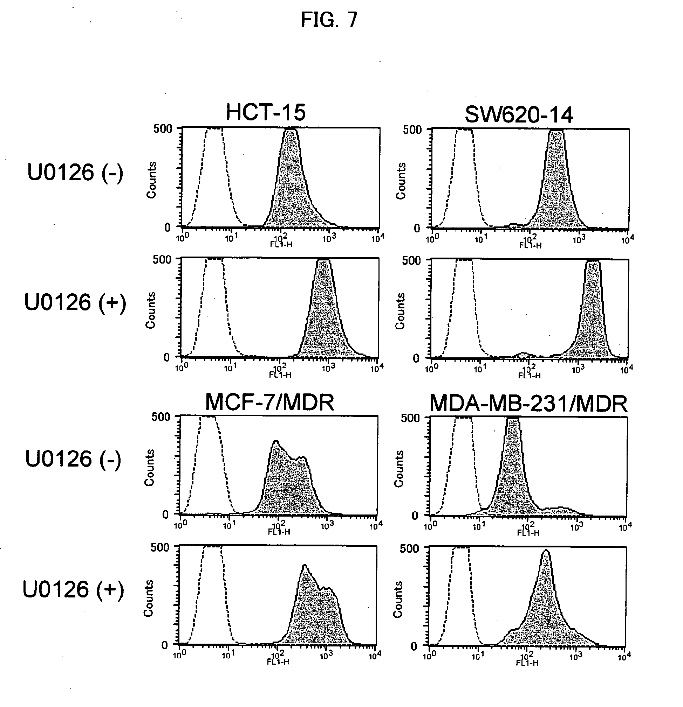

HCT-15 (1 x 10⁵/60 mm-dish), SW620-14 (2 x 10⁵/60 mm-dish), MCF-7/MDR (1 x 10⁵/60 mm-dish) and MDA-MB-231/MDR (2 x 10⁵/60 mm-dish) cells (these cells were obtained as described in EXAMPLE 5) were incubated for 72 hours in 10 µmol/L U0126-containing medium as described in EXAMPLE 5. In the control group, the cells were likewise incubated in U0126-free medium for 72 hours. After the cells were recovered, the cell number was counted and the cell suspension in the medium was prepared at 1 x 10⁵/mL. Rhodamine 123 (Sigma Inc.) was added to the cell suspension at a final concentration of 300 nmol/L. For control without Rhodamine 123 uptake, the medium alone was used. The cells were incubated at 37°C for 20 minutes to allow uptake of Rhodamine 123 into the cells. The cells were then centrifuged to recover them. The cells were washed twice with ice-cooled PBS. The cells were resuspended in isoflow and analyzed by FACS. By measuring the brightness of Rhodamine 123, the uptake of Rhodamine 123 into the cells can be determined by FACS. The results are shown in FIG 7.

As shown in FIG 7, it was confirmed that the uptake of fluorogenic substrate Rhodamine 123 of P-glycoprotein into cancer cells was increased.

### INDUSTRIAL APPLICABILITY

According to the present invention, the P-glycoprotein expression inhibitors can be provided. According to the present invention, the BCRP expression inhibitors can also be provided.

It is expected that the anticancer agent comprising the P-glycoprotein or BCRP expression inhibitor according to (1) above, which is reduced in resistance acquisition, can restore the medical efficacy of anticancer drugs even against drug-resistant cancers, thus leading to effective cancer chemotherapy. Furthermore, since the anticancer agent can suppress the resistance, the dosage of the anticancer agent can be reduced to permit cancer chemotherapy with minimized adverse effects. Moreover, the anticancer agent opens possibilities for cancer chemotherapy even for patients for whom treatment with anticancer drugs has not been effective due to congenital or acquired overexpression of P-glycoprotein or BCRP.

In addition, the present invention provides the method of preventing the resistance to an anticancer agent to enhance therapeutic effects of the anticancer agent against cancer, which comprises administering an effective dose of the P-glycoprotein or BCRP expression inhibitor according to (1) above and an effective dose of the anticancer agent.

The agent for preventing the resistance to an anticancer agent comprising the P-glycoprotein or BCRP expression inhibitor according to (1) above is administered for cancer which has acquired or will acquire the resistance to anticancer agents, whereby the efficacy of anticancer agents is restored to enable effective cancer chemotherapy.

The agent for treating cancer comprising a combination of the P-glycoprotein or BCRP expression inhibitor according to (1) and an anticancer agent is administered for cancer which has acquired the resistance to anticancer agents, which enables cancer chemotherapy effective in restoring the medical efficacy of anticancer agents. In addition, the anticancer agent can suppress the resistance and hence, the dosage of the anticancer agent can be reduced to permit cancer chemotherapy with minimized adverse effects. Furthermore, the anticancer agent opens possibilities for cancer chemotherapy even for patients for whom treatment with anticancer drugs has not been effective due to congenital or acquired overexpression of P-glycoprotein or BCRP.

Furthermore, the present invention can provide the method of preventing acquisition of the resistance to an anticancer agent to treat cancer, which comprises administering an effective dose of an anticancer agent comprising the P-glycoprotein or BCRP expression inhibitor according to (1) above.

According to the present invention, the P-glycoprotein expression inhibitor can be obtained by using the activity of inhibiting Ras, Raf, MEK, ERK or RSK as an indicator. Further according to the present invention, the BCRP expression inhibitor can be obtained by using the activity of inhibiting Ras, Raf, MEK, ERK or RSK as an indicator.

## Claims

1. A P-glycoprotein or BCRP expression inhibitor comprising a MAPK signaling inhibitor.

2. The P-glycoprotein or BCRP expression inhibitor according to claim 1, wherein the MAPK signaling inhibitor is a substance that inhibits the expression of a protein involved in MAPK signaling or a substance that inhibits the activity of a protein involved in MAPK signaling.

3. The P-glycoprotein or BCRP expression inhibitor according to claim 1 or 2, wherein the MAPK signaling inhibitor is one or at least two selected from a Ras inhibitor, a Raf inhibitor, a MEK inhibitor, an ERK inhibitor and a RSK inhibitor.

4. The P-glycoprotein or BCRP expression inhibitor according to any one of claims 1 to 3, wherein the MAPK signaling inhibitor is at least one selected from:
(a) a low molecular weight compound that inhibits the activity of a Ras protein, a Raf protein, a MEK protein, an ERK protein or a RSK protein;
(b) a low molecular weight compound that inhibits the expression of a Ras protein, a Raf protein, a MEK protein, an ERK protein or a RSK protein;
(c) siRNA or shRNA for a polynucleotide encoding a Ras protein, a Raf protein, a MEK protein, an ERK protein or a RSK protein; and,
(d) an antisense polynucleotide comprising the entire or part of a nucleotide sequence complementary or substantially complementary to a nucleotide sequence of a polynucleotide encoding a Ras protein, a Raf protein, a MEK protein, an ERK protein or a RSK protein.

5. A method of screening a P-glycoprotein or BCRP expression inhibitor using the activity of inhibiting Ras, Raf, MEK, ERK or RSK as an indicator.

6. An anticancer agent comprising the P-glycoprotein or BCRP expression inhibitor according to claim 1, which is reduced in resistance acquisition.

7. An agent for preventing the resistance to an anticancer agent comprising the P-glycoprotein or BCRP expression inhibitor according to claim 1.

8. The agent for preventing the resistance to an anticancer agent according to claim 7, wherein the MAPK signaling inhibitor is a substance that inhibits the expression of a protein involved in MAPK signaling or a substance that inhibits the activity of a protein involved in MAPK signaling.

9. The agent for preventing the resistance to an anticancer agent according to claim 7 or 8, wherein the MAPK signaling inhibitor is one or at least two selected from a Ras inhibitor, a Raf inhibitor, a MEK inhibitor, an ERK inhibitor and a RSK inhibitor.

10. An agent for treating cancer comprising a combination of the P-glycoprotein or BCRP expression inhibitor according to claim 1 and an anticancer agent.

11. The agent for treating cancer according to claim 10, wherein the MAPK signaling inhibitor is a substance that inhibits the expression of a protein involved in MAPK signaling or a substance that inhibits the activity of a protein involved in MAPK signaling.

12. The agent for treating cancer according to claim 10 or 11, wherein the MAPK signaling inhibitor is one or at least two selected from a Ras inhibitor, a Raf inhibitor, a MEK inhibitor, an ERK inhibitor and a RSK inhibitor.

13. The agent for treating cancer according to any one of claims 10 to 12, wherein the anticancer agent is selected from doxorubicin hydrochloride, daunomycin, epirubicin hydrochloride, an anthracycline, a vinca alkaloid and a taxane.

14. The method of preventing the expression of a P-glycoprotein or BCRP, which comprises administering an effective dose of a MAPK signaling inhibitor.

15. A method of preventing the expression of the P-glycoprotein or BCRP according to claim 4, wherein the MAPK signaling inhibitor is one or at least two selected from a Ras inhibitor, a Raf inhibitor, a MEK inhibitor, an ERK inhibitor and RSK inhibitor.

16. A method of treating cancer, which comprises administering an effective dose of an anticancer agent comprising the P-glycoprotein or BCRP expression inhibitor according to claim 1.

17. The method according to claim 16, wherein acquisition of resistance to the anticancer agent is reduced to treat cancer.

18. The method according to claim 16, wherein excretion of the anticancer agent from a target cancer cell of the anticancer agent is reduced to enhance the therapeutic effect on cancer.
